# EUROPÄISCHE PATENTSCHRIFT

(11) **EP 2 265 263 B1**
(45) Veröffentlichungstag und Bekanntmachung des Hinweises auf die Patenterteilung: **18.12.2013**
(21) Anmeldenummer: 09723543.6
(22) Anmeldetag: 26.01.2009
(51) Int. Cl.: A61K 36/81, A61K 31/05, A61K 31/353, A61K 31/375, A61K 31/4415, A61K 31/714, A61K 33/04, A23G 4/06, A23G 4/12, A61K 45/06, A61K 9/68

(54) **KAUZUSAMMENSETZUNG UND DEREN VERWENDUNG**
CHEWING COMPOSITION AND THE USE THEREOF
COMPOSITION À MÂCHER ET UTILISATION

(30) Priorität: 19.03.2008 DE 102008015101
(43) Veröffentlichungstag der Anmeldung: 29.12.2010
(73) Patentinhaber: Maria Clementine Martin Klosterfrau Vertriebsgesellschaft mbH, 50670 Köln (DE)
(72) Erfinder: VESTWEBER, Anna-Maria, 51399 Burscheid (DE); SCHLÜTER, Andreas, 29439 Lüchow (DE)
(74) Vertreter: Von Rohr
(86) Internationale Anmeldenummer: PCT/EP2009/000486
(87) Internationale Veröffentlichungsnummer: WO 2009/115160

(56) Entgegenhaltungen:
- EP-A- 1 304 048
- WO-A-00/25612
- WO-A-81/02090
- WO-A-2007/053096
- US-A- 5 092 352
- DATABASE WPI Week 199730 Thomson Scientific, London, GB; AN 1997-320234 XP002557208 & CN 1 105 215 A (SUN L) 19. Juli 1995 (1995-07-19)
- DATABASE WPI Week 200409 Thomson Scientific, London, GB; AN 2004-083793 XP002557209 & CN 1 451 412 A (LI G) 29. Oktober 2003 (2003-10-29)

## Beschreibung

Die vorliegende Erfindung betrifft den Bereich der Raucherentwöhnung, die Erleichterung der Aufgabe des Rauchens, die Behandlung einer Tabak- und/oder Nikotinabhängigkeit bzw. die Linderung von Nikotinentzugssymptomen.

Insbesondere betrifft die vorliegende Erfindung eine Kauzusammensetzung in Form eines Kaugummis, welche insbesondere zur Raucherentwöhnung eingesetzt werden soll.

Des weiteren betrifft die vorliegende Erfindung eine Verpackungseinheit, welche mindestens eine, insbesondere eine Vielzahl von Dosiereinheiten der erfindungsgemäßen Kauzusammensetzung enthält.

Schließlich betrifft die vorliegende Erfindung die Verwendung der Zusammensetzung zur Raucherentwöhnung, zur Erleichterung der Aufgabe des Rauchens, zur Behandlung einer Tabak- und/oder Nikotinabhängigkeit bzw. zur Linderung von Nikotinentzugssymptomen.

Das Rauchen von Tabakwaren bzw. Raucherzeugnissen, wie Zigaretten, Zigarillos, Zigarren und Pfeife, stellt eine hohe gesundheitliche Belastung für den Konsumenten dar. Insbesondere ist der Konsum von Rauchwaren auf Tabakbasis mit einem hohen Abhängigkeitspotential verbunden, was durch eine Vielzahl von im Tabakrauch enthaltenen Substanzen, darunter Nikotin, hervorgerufen wird.

Zudem weist der bei der Verbrennung von Tabak entstehende Tabakrauch eine außerordentlich hohe Toxizität auf, da dieser eine Vielzahl chemischer Substanzen bzw. chemischer Verbrennungsprodukte enthält, die eine mehr oder weniger starke Toxizität und/oder Karzinogenität aufweisen. Zigarettenrauch enthält insgesamt bis zu 12.000 verschiedene chemische Verbindungen in allen drei Aggregatzuständen, von denen über 2.000 als Giftstoffe bekannt sind. Die Freisetzung bzw. Entstehung der toxisch bzw. karzinogen wirkenden Substanzen ist im Zusammenhang mit den komplexen chemischen Prozessen, die beim Tabakrauchen ablaufen, zu sehen.

So liegen beim Tabakrauchen im wesentlichen drei unterschiedliche chemische Prozesse vor: Zum einen verbrennt der Tabak in oxidierender Atmosphäre, wobei vorrangig Redoxreaktionen in der sogenannten Glutzone ablaufen. Zum anderen verschwelt der Tabak insbesondere in und hinter dem Glutkegel unter vorrangig reduzierender Atmosphäre, was eine unvollständige Verbrennung sowie eine thermische Zersetzung bzw. Pyrolyse zahlreicher Substanzen bewirkt. Hierdurch entstehen schädliche ungesättigte Verbindungen sowie toxische Kondensations- und Polymerisationsprodukte. Schließlich verdampft und destilliert der flüchtige Anteil des Tabaks, wobei niedrigsiedende Stoffe direkt in den Rauch übergehen, und der beim Verrauchen freiwerdende Wasserdampf reißt nach dem Prinzip einer Wasserdampfdestillation bzw. -extraktion weitere Schadstoffe sowie Nikotin und ätherische Öle mit sich.

Der resultierende Tabakrauch ist ein Aerosol, dessen Gasphase zahlreiche flüchtige organische Substanzen und in Suspension eine Partikelphase enthält, welche aus Feststoffen und Flüssigkeiten zusammengesetzt ist, die bei der Verbrennungstemperatur des Tabaks mitunter verdampfen. So kann die Gasphase pro Kubikzentimeter Tabakrauch zwei bis drei Milliarden feinste Tröpfchen aus der Partikelphase enthalten.

Wie zuvor angeführt, enthält der in Form eines Aerosols vorliegende Tabakrauch eine Vielzahl von toxischen Substanzen, wobei diesbezüglich Benzol, Blausäure, Methanal bzw. Formaldehyd und Nitrosamine als einige der wichtigsten Vertreter zu nennen sind. Zu den toxischen Substanzen können auch reizende Substanzen gezählt werden, wobei es sich in bezug auf die Tabakverbrennung insbesondere um stickstoffhaltige Verbindungen, wie Ammoniak (NH₃) und Stickstoffoxide, wie NO und N₂O₄, handelt. Stickstoffoxide stehen zudem unter dem Verdacht, krebserregend zu sein. Weiterhin enthält der Zigarettenrauch eine Vielzahl bluttoxischer Substanzen, wobei Kohlenstoffmonoxid und gleichermaßen Blausäure zu nennen sind. Darüber hinaus umfaßt der Tabakrauch auch zahlreiche neurotoxische Substanzen, wobei Nikotin als ein wesentlicher Vertreter zu nennen ist. Tabakrauch umfaßt auch eine Vielzahl karzinogener Substanzen, wie beispielsweise Benzol, 3,4-Benzopyren. Schließlich enthält Tabakrauch auch freie Radikale, wie Sauerstoffradikale, welchen gleichermaßen eine hohe Karzinogenität zugesprochen wird.

Darüber hinaus umfaßt die Partikelphase des Tabakrauchs, welche synonym auch als "Teer" bzw. Kondensat bezeichnet wird, weitere Schadstoffe, welche mitunter auch eine Verlangsamung des Selbstreinigungssystems der Bronchien bewirken. Dies kann dazu führen, daß bei regelmäßiger Teer- und Kondensatzufuhr eine chronische Bronchitis ("Raucherhusten") entwickelt wird, welche auch nach einem sofortigen völligen Beenden des Rauchens unter Umständen noch monate- bis jahrelang anhalten kann.

Das Einatmen von Tabakrauch ist somit ein gesicherter Risikofaktor für verschiedene Arten von Krebs, Lungenkrankheiten sowie Krankheiten des Herz/KreislaufSystems. Denn die Schadstoffaufnahme beim Tabakrauchen ist enorm, was anhand der folgenden Beispielrechnung veranschaulicht werden kann. Bei einem Konsum von zwanzig Zigaretten täglich über einen Zeitraum von zwanzig Jahren werden insgesamt sechs Kilogramm Rauchstaub und jährlich etwa 200 ml Teer aufgenommen. Diese Art von Rauchvergiftung verkürzt - statistisch gesehen - die Lebensdauer eines Rauchers um etwa sechs Jahre. Statistiken zeigen weiterhin, daß beispielsweise im Jahr 2000 der Konsum von Tabakrauchwaren weltweit für jeden zehnten Todesfall unter Erwachsenen verantwortlich war. In den Vereinigten Staaten von Amerika und Westeuropa sind sogar fast 25 % der Todesfälle in der Personengruppe mit einem Alter von 30 bis 69 Jahren Folge des Rauchens.

Auf der anderen Seite belegen Studien aber auch, daß eine Raucherentwöhnung bzw. eine Aufgabe des Rauchens zu einer signifikanten Verbesserung des Gesundheitszustands und zu einer Erhöhung der Lebenserwartung führen. So konnte gezeigt werden, daß eine Einstellung des Rauchens vor dem 35. Lebensjahr dazu führt, daß die Lebenserwartung mit zunehmender Zeit des Nichtrauchens nahezu auf diejenige eines lebenslangen Nichtrauchers ansteigt, wobei auch die Lebensqualität und die Krankheitshäufigkeit denen eines Nichtrauchers entsprechen. Auch eine Einstellung des Rauchens im mittlern Alter kann noch zu einer signifikanten Erhöhung der Lebenserwartung führen.

Der Raucherentwöhnung kommt somit insbesondere im Sinne der Verbesserung des persönlichen Wohlbefindens, der Gesundheit und der Lebensqualität eine hohe Relevanz zu.

Problematisch sind jedoch die mit der Raucherentwöhnung oftmals einhergehenden Begleiterscheinungen: Denn durch das plötzliche Absetzen von Tabakraucherzeugnissen nach längerer Zeit der Anwendung mit einhergehendem Absetzen der Zufuhr von Nikotin ist mit typischen Entzugssymptomen zu rechnen. Hierzu zählen beispielsweise Dysphorie oder Schwermütigkeit, Schlaflosigkeit, Zorn, Angst, Konzentrationsschwierigkeiten, Ruhelosigkeit oder Ungeduld, niedrige Herzfrequenz sowie gesteigerter Appetit bzw. Gewichtszunahme. Zudem stellt das starke Verlangen nach Nikotin ein weiteres klinisch relevantes Symptom beim Tabakentzug dar. Oftmals führen diese mitunter stark ausgeprägten Symptome, die auch über eine längere Zeit andauern können, zu einem vorzeitigen Abbruch einer Raucherentwöhnung.

Im Stand der Technik sind bislang verschiedene Ansätze realisiert worden, die eine Raucherentwöhnung bzw. eine Aufgabe des Rauchens unterstützen sollen:
So betrifft die DE 698 11 378 T2 eine Zusammensetzung zur Raucherentwöhnung, wobei die Zusammensetzung eine Kombination eines speziellen Nikotinrezeptor-Antagonisten, nämlich Mecamylamin, und ein Antidepressivum, nämlich Bupropion, umfaßt. Nachteilig bei einer derartigen Zusammensetzung sind jedoch die mitunter auftretenden Nebenwirkungen. Zudem werden auftretende Entzugserscheinungen vorrangig nur symptomatisch behandelt.

Weiterhin wird im Stand der Technik darauf abgestellt, eine Raucherentwöhnung auf Basis einer Applikation bzw. Verabreichung von Nikotin bzw. Nikotinderivaten zu realisieren.

So sind im Stand der Technik transdermale Nikotinpflaster bekannt, welche über einen speziellen Aufbau des Pflasters zu einer kontrollierten Abgabe von Nikotin führen sollen. Ein Verfahren zur Herstellung eines derartigen Pflasters ist beispielsweise in der DE 689 29 533 T2 beschrieben. Nachteilig bei einer derartigen Applikationsform ist jedoch die Tatsache, daß einerseits die Nikotinfreisetzung und damit einhergehend der Verlauf der Nikotinkonzentration im Blutplasma nicht immer optimal ist und darüber hinaus andererseits Unverträglichkeitsreaktionen am Applikationsort des Pflasters resultieren können. Zudem resultiert oftmals die Problematik, daß es sich bei der Applikation mittels eines Pflasters in bezug auf den zu entwöhnenden Raucher um einen lediglich passiven Vorgang handelt, so daß der Raucher sozusagen keine "Ersatzhandlung" für den aktiven körperlichen Vorgang des Rauchens eines Tabakerzeugnisses hat. Demnach ist die Rückfallquote bei einer derartigen Applikationsform mitunter recht hoch.

Gleichermaßen sind im Stand der Technik Nikotin enthaltende Tabletten bekannt, welche oral appliziert werden. Weiterhin sind im Stand der Technik zur Raucherentwöhnung auch Kaugummis bekannt, welche Nikotin bzw. Nikotinderivate als Reinsubstanz bzw. in Form der isolierten Substanz enthalten. Nachteilig bei den zuvor genannten Applikationsformen ist insbesondere, daß die Freisetzung des Nikotins aus der Tablette bzw. aus dem Kaugummi mitunter unkontrolliert bzw. zu schnell erfolgt, was zu einem ungünstigen Verlauf der Nikotinkonzentration im Blutplasma führt, was jedoch unerwünscht ist. Zudem weisen die vorgenannten Applikationsformen auf Basis einer Tablette bzw. eines Kaugummis nicht immer als angenehm empfundene organoleptische Eigenschaften auf.

Weiterhin betrifft die EP 1 304 048 A1 eine Zusammensetzung, um einer Person dabei zu helfen, mit dem Rauchen aufzuhören, wobei die Zusammensetzung eine Tabak/Antioxidans-Formulierung aufweist, wobei das Antioxidans ausgewählt ist aus der Gruppe von Aminosäuren, Enzymen, Chemikalien, Hormonen, Pflanzenextrakten und Proteinen. Zudem soll die Tabakkomponente in Pulverform eingesetzt werden.

Insgesamt führen die im Stand der Technik vorgesehenen Ansätze zur Raucherentwöhnung oftmals nicht zu einem ausreichenden Therapieerfolg bzw. zu unerwünschten Nebenwirkungen, wobei insbesondere in bezug auf oral zu applizierende nikotinhaltige Präparate des Standes der Technik die organoleptischen Eigenschaften nicht immer optimal sind.

Vor diesem Hintergrund liegt der vorliegenden Erfindung somit die Aufgabe zugrunde, eine Zusammensetzung bereitzustellen, welche sich zur Raucherentwöhnung bzw. zur Erleichterung der Aufgabe des Rauchens bzw. zur Behandlung von Tabak- und Nikotinabhängigkeit bzw. zur Linderung von Nikotinentzugssymptomen eignet und welche die zuvor geschilderten Nachteile des Standes der Technik zumindest weitgehend vermeidet oder zumindest abschwächt. Dabei soll insbesondere eine verbesserte Freisetzung der Wirksubstanz auf Basis von Nikotin bei gleichzeitig verbesserten organoleptischen Eigenschaften gewährleistet sein.

Die Anmelderin hat nunmehr in überraschender Weise herausgefunden, daß die zuvor gestellte Aufgabe dadurch gelöst werden kann, daß man eine Kauzusammensetzung bereitstellt, welche eine Kaugummigrundmasse und einen nikotinhaltigen Kautabak aufweist.

Gegenstand der vorliegenden Erfindung ist somit eine Kauzusammensetzung in Form eines Kaugummis, insbesondere zur Raucherentwöhnung, wobei die Kauzusammensetzung neben
a) 20 Gew.-% bis 95 Gew.-% einer Kaugummigrundmasse
b) 5 Gew.-% bis 60 Gew.-% eines nikotinhaltigen Kautabaks enthält, wobei die Kauzusammensetzung zudem
c) mindestens ein Phytoalexin in Mengen von 0,1 Gew.-% bis 10 Gew.-%,
d) Ascorbinsäure (Vitamin C) in Mengen von 0,1 Gew.-% bis 10 Gew.-%,
e) mindestens ein Polyphenol in Mengen von 0,2 Gew.-% bis 10 Gew.-%,
f) Selen in Mengen von 1·10⁻⁷ Gew.-% bis 1·10⁻² Gew.-% und
g) mindestens ein Vitamin B in Mengen von 0,5·10⁻⁷ Gew.-% bis 0,5·10⁻² Gew.-% enthält, wobei die Mengenangaben jeweils auf die Kauzusammensetzung bezogen sind.

Der Begriff "Kauzusammensetzung", wie er im Rahmen der vorliegenden Erfindung verwendet wird, ist sehr umfassend zu verstehen. So kann die erfindungsgemäße Kauzusammensetzung in Form eines Medizinproduktes, Nahrungsergänzungsmittels, homöopathisches Mittel oder als pharmazeutische Zusammensetzung bzw. Zubereitung bzw. als tabakhaltiges Produkt oder dergleichen vorliegen bzw. eingesetzt werden.

Die Anmelderin hat überraschenderweise herausgefunden, daß durch die erfindungsgemäße Kauzusammensetzung, welche neben einer Kaugummigrundmasse einen nikotinhaltigen Kautabak enthält, die zuvor gestellte Aufgabenstellung in effizienter Weise gelöst wird und ein wirksames Produkt zur Raucherentwöhnung bereitgestellt wird.

Diesbezüglich hat die Anmelderin in völlig überraschender Weise herausgefunden, daß durch die erfindungsgemäße Inkorporation eines nikotinhaltigen Kautabaks in eine Kaugummigrundmasse die Freisetzung von Nikotin während des Kauens der erfindungsgemäßen Kauzusammensetzung dahingehend optimiert ist, daß zum einen ein zu schnelles Ansteigen der Konzentration von Nikotin im Blutplasma verhindert wird und zum anderen die Konzentration von Nikotin im Blutplasma über einen längeren Zeitraum konstant bzw. auf hohem Niveau verbleibt, wie nachfolgend noch im Rahmen der Ausführungsbeispiele ausführlich beschrieben wird. Hierdurch werden die mit dem der Raucherentwöhnung einhergehenden nikotinbedingten Entzugserscheinungen in besonders effektiver Weise unterbunden bzw. minimiert.

Im Rahmen der oralen Applikation der erfindungsgemäßen Kauzusammensetzung bzw. beim Kauen der erfindungsgemäßen Kauzusammensetzung im Mund findet somit gewissermaßen eine kontrollierte bzw. verzögerte bzw. retardierte Freisetzung von Nikotin statt, was zu dem zuvor beschriebenen Verlauf der Blutplasmakonzentration von Nikotin mit dem weniger starken Anfangsanstieg und der im zeitlichen Verlauf auf hohem Niveau verbleibenden höheren Konzentration von Nikotin im Blutplasma einhergeht.

In diesem Zusammenhang kann der Freisetzungsmechanismus von Nikotin aus der erfindungsgemäßen Zusammensetzung - ohne sich auf diese Theorie festlegen zu wollen - derart verstanden werden, daß das Nikotin gewissermaßen in kontrollierter bzw. verzögerter Weise aus der durch den nikotinhaltigen Kautabak selbst und der Kaugummigrundmasse gebildeten Matrix der Kauzusammensetzung mit einer gewissen Verzögerung und über einen längeren Zeitraum freigesetzt wird. Die erfindungsgemäße Kauzusammensetzung weist somit sozusagen eine in bezug auf die Freisetzung von Nikotin optimierte Matrix auf.

Vor diesem Hintergrund eignet sich die erfindungsgemäße Kauzusammensetzung in hervorragender Weise zur Raucherentwöhnung, insbesondere da Nikotin kontinuierlich über einen längeren Zeitraum abgegeben wird und ein schnelles Anfluten des Nikotins im Blutplasma mit anschließendem schnellen Abklingen, wie es beim Rauchen einer Zigarette auftritt und zu unerwünschten Effekten bzw. Nebenwirkungen führen kann, vermieden wird.

Die erfindungsgemäße Kauzusammensetzung eignet sich - wie zuvor angeführt - insbesondere aufgrund der kontrollierten und kontinuierlichen Freisetzung von Nikotin während des Kauens in besonderer Weise zur Raucherentwöhnung, da durch die gezielte Applikation von Nikotin die zuvor geschilderten Entzugserscheinungen gelindert werden können und so den Rauchern geholfen wird, das Rauchen aufzugeben. Dabei ist die Aufnahme von Nikotin auf Basis der erfindungsgemäßen Zusammensetzung im Vergleich zu der durch das Rauchen einer Zigarette bedingten Nikotinaufnahme geringer, wobei insbesondere der bei dem Rauchen einer Zigarette vorliegende starke Anfangsanstieg der Nikotinkonzentration im Blutplasma verhindert wird. Die erfindungsgemäße Kauzusammensetzung führt somit bei ihrer Applikation zu deutlich verminderten Nebenwirkungen.

Darüber hinaus verfügt die erfindungsgemäße Kauzusammensetzung über hervorragende organoleptische Eigenschaften, die insbesondere gegenüber Kaugummis des Standes der Technik mit reinem Nikotin deutlich verbessert sind, so daß das Konsumieren bzw. das Kauen der erfindungsgemäßen Kauzusammensetzung als angenehm empfunden wird. Durch den nikotinhaltigen Kautabak wird bei der erfindungsgemäßen Kauzusammensetzung das "Kaugefühl" deutlich verbessert. Durch die gezielte Verwendung eines nikotinhaltigen Kautabaks werden während des Kauvorgangs zudem tabakspezifische Geschmacks- bzw. Aromastoffe freigesetzt, so daß die erfindungsgemäße Kauzusammensetzung einen für den zu entwöhnenden Raucher angenehmen (Tabak-)Geschmack aufweist. Durch die gezielte Zugabe weiterer Geschmacks- bzw. Aromastoffe kann die erfindungsgemäße Zusammensetzung zusätzlich hinsichtlich ihres Geschmacks ausgerichtet bzw. eingestellt werden.

Zudem weist die erfindungsgemäße Kauzusammensetzung den entscheidenden Vorteil auf, daß ihre Verabreichung gewissermaßen unter aktiver Beteiligung des zu entwöhnenden Rauchers erfolgt, da die erfindungsgemäße Kauzusammensetzung sozusagen aktiv über einen längeren Zeitraum gekaut wird, so daß gewissermaßen eine motorische "Ersatzbeschäftigung" bzw. "Kompensationshandlung" geschaffen wird mit der Folge, daß die mit dem Rauchen einhergehenden (motorische) Verhaltensmuster gewissermaßen verdrängt bzw. überlagert werden, wodurch auch das durch Gewohnheitshandlungen hervorgerufene Rauchbedürfnis verringert wird.

Die erfindungsgemäße Kauzusammensetzung ermöglicht somit eine Verabreichung von Nikotin in definierter Menge und mit definiertem Zeitverlauf, so daß die mit dem Rauchen einhergehenden Entzugserscheinungen signifikant gelindert werden und somit die Entwöhnung insgesamt leichter fällt. Die erfindungsgemäße Zusammensetzung kann dabei in variabler Weise appliziert werden. So kann - wie nachfolgend noch angeführt - die erfindungsgemäße Zusammensetzung zu Beginn des Einstellens bzw. Absetzens des Rauchwarenkonsums häufiger bzw. mit höherem Nikotingehalt angewendet werden, so daß höhere Nikotinkonzentrationen im Blutplasma eingestellt werden. Im weiteren Verlauf nach Beendigung des Rauchens sollte die erfindungsgemäße Kauzusammensetzung weniger häufig bzw. mit geringerem Nikotingehalt verabreicht bzw. konsumiert werden, um im zeitlichen Verlauf der Entwöhnung eine kontinuierliche Verminderung der Nikotinverabreichung zu ermöglichen, bis eine vollständige Entwöhnung und somit ein Einstellen des Suchtverhaltens resultiert, wonach die Verabreichung der erfindungsgemäßen Kauzusammensetzung eingestellt werden kann.

Bei allen nachstehend genannten relativen bzw. prozentualen gewichtsbezogenen Mengenangaben ist zu beachten, daß diese im Rahmen der erfindungsgemäßen Zusammensetzung vom Fachmann derart auszuwählen sind, daß sie sich in der Summe unter Einbeziehung der Komponenten bzw. Inhaltsstoffe bzw. Zusatzstoffe bzw. Bestandteile bzw. Exzipienten, insbesondere wie nachfolgend definiert, stets um 100 Gew.-% ergänzen. Dies versteht sich für den Fachmann aber von selbst. Im übrigen gilt, daß der Fachmann anwendungsbezogen oder einzelfallbedingt von den nachfolgend aufgeführten Mengenangaben abweichen kann, ohne daß er den Rahmen der vorliegenden Erfindung verläßt.

Was die erfindungsgemäße Kauzusammensetzung anbelangt, so liegt diese in Form eines Kaugummis vor. Dies wird maßgeblich durch die Verwendung einer Kaugummigrundmasse ermöglicht.

In diesem Zusammenhang ist es erfindungsgemäß bevorzugt, daß der Kautabak in die Kaugummigrundmasse eingearbeitet und/oder eingebettet ist, so daß gewissermaßen ein Gemisch aus Kaugummigrundmasse und nikotinhaltigem Kautabak resultiert. In erfindungsgemäß bevorzugter Art und Weise ist der Kautabak in zumindest im wesentlichen homogener Verteilung in die Kaugummigrundmasse eingearbeitet bzw. eingebettet. Mit anderen Worten ist der nikotinhaltige Kautabak gleichmäßig in die Kaugummigrundmasse eingearbeitet, so daß gleichermaßen eine gleichmäßige Inkorporierung des nikotinhaltigen Kautabaks in die Kaugummigrundmasse vorliegt. Der Kautabak ist sozusagen in die aus der Kaugummigrundmasse gebildete Matrix eingelagert. Dies führt insbesondere zu einer Verbesserung des Freisetzungsverhaltens von Nikotin beim Kauen der erfindungsgemäßen Kauzusammensetzung.

Besonders gute Ergebnisse hinsichtlich der organoleptischen Eigenschaften lassen sich realisieren, wenn die erfindungsgemäße Kauzusammensetzung ausgebildet ist derart, daß der Kautabak in zerkleinerter bzw. feinverteilter Form, insbesondere pulverisiert oder vermahlen, in die Kaugummigrundmasse eingearbeitet und/oder eingebettet ist. Hierdurch wird zudem die Freisetzung der Inhaltsstoffe aus dem Kautabak - darunter auch Nikotin - verbessert.

In diesem Zusammenhang kann es erfindungsgemäß vorgesehen sein, daß der Kautabak eine mittlere Partikel- und/oder Fasergröße von 0,001 µm bis 5 mm, insbesondere 0,01 µm bis 3 mm, vorzugsweise 0,1 µm bis 2 mm, bevorzugt 1 µm bis 1 mm, aufweist. Hierdurch werden besonders positive organoleptische Eigenschaften erreicht, so daß das Kaugefühl stets angenehm ist.

Was den im Rahmen der vorliegenden Erfindung eingesetzten Kautabak als solchen anbelangt, so sollte dieser einen Nikotingehalt von 0,01 Gew.-% bis 2 Gew.-%, insbesondere 0,05 Gew.-% bis 1,5 Gew.-%, vorzugsweise 0,1 Gew.-% bis 1,25 Gew.-%, bevorzugt 0,2 Gew.-% bis 1 Gew.-%, bezogen auf den Kautabak, aufweisen.

Was die absoluten Nikotinmengen in bezug auf die Kauzusammensetzung als solche nach der Erfindung anbelangt, so kann die erfindungsgemäße Kauzusammensetzung eine Nikotinmenge von 0,1 mg bis 20 mg, insbesondere 0,5 mg bis 15 mg, vorzugsweise 0,75 mg bis 10 mg, bevorzugt 1 mg bis 5 mg, bezogen auf eine Dosiereinheit der Kauzusammensetzung, insbesondere bezogen auf einen einzelnen Kaugummi, aufweisen. In erfindungsgemäß besonders bevorzugter Weise kann die Kauzusammensetzung insbesondere eine Nikotinmenge von etwa 2 mg, bezogen auf eine Dosiereinheit der Kauzusammensetzung, insbesondere bezogen auf einen einzelnen Kaugummi, aufweisen. Durch die gezielte Einstellung der Nikotinmenge in der erfindungsgemäßen Kauzusammensetzung - welche beispielsweise anhand der Menge und der Art des eingesetzten Kautabaks erfolgen kann - kann somit die zu verabreichende Nikotindosis eingestellt werden. So können beispielsweise hinsichtlich des Beginns einer Raucherentwöhnung Kauzusammensetzungen mit höherem Nikotingehalt bereitgestellt werden, wohingegen beispielsweise zum Ende einer durchzuführenden Raucherentwöhnung einhergehend mit der zunehmenden Entwöhnung des Rauchers erfindungsgemäße Kauzusammensetzungen mit geringerer Nikotinkonzentration bereitgestellt bzw. verabreicht werden können.

Der Begriff "Nikotin", wie er im Rahmen der vorliegenden Erfindung verwendet wird, umfaßt neben Nikotin an sich auch Derivate des Nikotins bzw. gleichwirkende Substanzen auf Nikotinbasis.

Was die Zerkleinerung des erfindungsgemäßen Kautabaks und seine Inkorporierung in die Kaugummigrundmasse der erfindungsgemäßen Kauzusammensetzung anbelangt, so kann dies in dem Fachmann an sich bekannter Art und Weise erfolgen. So kann die Zerkleinerung beispielsweise mittels Vermahlen, Pulverisieren, Zerschneiden oder mittels Verreiben erfolgen. Das Einarbeiten bzw. die Inkorporierung des erfindungsgemäß eingesetzten nikotinhaltigen Kautabaks in die Kaugummigrundmasse kann beispielsweise durch Vermengen, Verkneten oder dergleichen erfolgen. Was die erfindungsgemäße Kauzusammensetzung weiterhin anbelangt, so kann es erfindungsgemäß vorgesehen sein, daß die diesbezügliche Dosiereinheit, insbesondere der Kaugummi, mit dem Fachmann an sich bekannten Mitteln beschichtet ist. Als Beschichtungsmittel kann in dem Fachmann bekannter Weise beispielsweise Mannitol eingesetzt werden. Die diesbezüglich einsetzbaren Beschichtungsverfahren sind dem Fachmann gleichermaßen bekannt.

Bei der erfindungsgemäßen Kauzusammensetzung handelt es sich insgesamt - aufgrund der erfindungsgemäß vorgesehenen Maßnahmen - um eine angenehme und wohlschmeckende Kauzusammensetzung, insbesondere in Form eines Kaugummis, insbesondere zur Raucherentwöhnung.

Was den erfindungsgemäß eingesetzten nikotinhaltigen Kautabak anbelangt, so sollte es sich hierbei um einen Kautabak handeln, welcher einen möglichst niedrigen Nitrosamingehalt aufweist. So sollte der erfindungsgemäß eingesetzte Kautabak einen Nitrosamingehalt von höchstens 5 µg Nitrosamin(e) / g Kautabak, insbesondere höchstens 2 µg Nitrosamin(e) / g Kautabak, vorzugsweise höchstens 1 µg Nitrosamin(e) / g Kautabak, bevorzugt höchstens 0,5 µg Nitrosamin(e) / g Kautabak, besonders bevorzugt höchstens 1 µg Nitrosamin(e) / g Kautabak, aufweisen. Gemäß einer erfindungsgemäß besonders bevorzugten Ausführungsform sollte der eingesetzte nikotinhaltige Kautabak zumindest im wesentlichen nitrosaminfrei ausgebildet sein. Denn bei den Nitrosaminen handelt es sich - wie zuvor angeführt - um karzinogen wirkende Substanzen, so daß deren Menge in der erfindungsgemäßen Kauzusammensetzung möglichst gering sein sollte.

Im Rahmen der vorliegenden Erfindung einsetzbare Kautabake sind dem Fachmann als solche bekannt und sind vor dem Hintergrund der zuvor angeführten erfindungsgemäß bevorzugten Spezifikationen auszuwählen. Im allgemeinen handelt es sich bei einem Kautabak laut Tabaksteuergesetz vom 13. Dezember 1979 (BGBI. I, Seite 2118) um "Tabak in Rollen, Stangen, Streifen, Würfeln oder Platten, der so zubereitet ist, daß er sich nicht zum Rauchen, sondern zum Kauen eignet". In nichtbeschränkender Weise kann beispielsweise handelsüblich erhältlicher Kautabak mit der Bezeichnung "*Oliver Twist*" eingesetzt werden, welcher von der Firma House of Oliver Twist A/S, Dänemark, hergestellt und von der Firma Kohlhase & Kopp importiert wird. Zudem kann der erfindungsgemäß verwendete Kautabak mit Tabakextrakten und aromatischen Zusätzen, wie Süßholz oder Honig derart gesüßt sein, daß die resultierenden organoleptischen Eigenschaften der erfindungsgemäßen Kauzusammensetzung weiter verbessert werden. Zudem kann der erfindungsgemäß eingesetzte Kautabak beispielsweise Glycerin als Feuchthaltemittel, *Gummi arabicum* als Verdickungsmittel und Eisen(II)-Sulfat als Farbstoff enthalten.

Die erfindungsgemäße Kauzusammensetzung sollte den Kautabak in Mengen von 5 Gew.-% bis 60 Gew.-%, insbesondere 10 Gew.-% bis 50 Gew.-%, vorzugsweise 20 Gew.-% bis 40 Gew.-%, bevorzugt 25 Gew.-% bis 35 Gew.-%, bezogen auf die Kauzusammensetzung, aufweisen.

Die absolute Menge an in der erfindungsgemäßen Kauzusammensetzung vorhandenem Kautabak kann 50 mg bis 1.000 mg, insbesondere 200 mg bis 800 mg, vorzugsweise 300 mg bis 700 mg, bevorzugt 400 mg bis 600 mg, bezogen auf eine Dosiereinheit der Kauzusammensetzung, insbesondere bezogen auf einen einzelnen Kaugummi, betragen.

Was die erfindungsgemäße Kaugummigrundmasse anbelangt, so sollte diese zumindest im wesentlichen wasserunlöslich, insbesondere in Speichel zumindest im wesentlichen unlöslich, und/oder zumindest im wesentlichen unverdaulich sein.

Im Rahmen der vorliegenden Erfindung können diesbezüglich eine Vielzahl an dem Fachmann an sich bekannten Kaugummigrundmassen eingesetzt werden, wobei diesbezüglich gewährleistet sein sollte, daß der erfindungsgemäß eingesetzte Kautabak homogen bzw. gleichmäßig in die Kaugummigrundmasse eingearbeitet werden kann. Im allgemeinen handelt es sich bei der erfindungsgemäß verwendeten Kaugummigrundmasse in bevorzugter Weise um eine beim Kauen plastisch werdende Kaugummigrundmasse.

In erfindungsgemäß bevorzugter Art und Weise enthält die Kaugummigrundmasse natürliche Polymere, insbesondere Kautschuk, vorzugsweise in Form von Latex, Crepe oder Sheets, und/oder Polyole. Im allgemeinen kann die Kaugummigrundmasse aus Pflanzenexsudaten stammende konsistenzgebende Polymere bzw. Pflanzenexsudate an sich enthalten. Die erfindungsgemäß eingesetzte Kaugummigrundmasse kann gleichermaßen synthetische Polymere, insbesondere synthetische Thermoplaste, vorzugsweise Polyvinylether, insbesondere Poly(ethylvinylether) und/oder Poly(isobutylvinylether), und/oder Polyisobutene, enthalten.

Im Rahmen der vorliegenden Erfindung ist es gleichermaßen möglich, daß die Kaugummigrundmasse als solche zusätzlich zu den vorgenannten struktur- bzw. konsistenzgebenden Stoffen weitere Füllstoffe enthält, wie Aluminiumoxid, Kieselsäure oder Cellulose. Gleichermaßen kann die erfindungsgemäß verwendete Kaugummigrundmasse an sich Geschmacks- bzw. Aromastoffe enthalten. Zu weitergehenden Einzelheiten in bezug auf den Begriff des Kaugummis kann auf das diesbezügliche Stichwort in Römpp Chemielexikon, 10. Auflage, Band 3, Georg Thieme Verlag Stuttgart/New York, verwiesen werden.

Gemäß einer erfindungsgemäß besonders bevorzugten Ausführungsform kann eine Kaugummigrundmasse beispielsweise auf Basis von Pharmagum·, insbesondere Pharmagum· M, eingesetzt werden, welche durch die Firma SPI Pharma, Michigan, USA, hergestellt wird und in der Bundesrepublik Deutschland durch die Firma Lehmann & Voss & Co., Hamburg, vertrieben wird.

In diesem Zusammenhang sollte die Kauzusammensetzung nach der Erfindung die Kaugummigrundmasse in Mengen von 20 Gew.-% bis 95 Gew.-%, insbesondere 30 Gew.-% bis 90 Gew.-%, vorzugsweise 40 Gew.-% bis 80 Gew.-%, bevorzugt 50 Gew.-% bis 70 Gew.-%, bezogen auf die Kauzusammensetzung, aufweisen.

Was die absolute Menge der Kaugummigrundmasse in der erfindungsgemäßem Kauzusammensetzung anbelangt, so kann die erfindungsgemäße Kauzusammensetzung die Kaugummigrundmasse in Mengen von 100 mg bis 5.000 mg, insbesondere 200 mg bis 3.000 mg, vorzugsweise 300 mg bis 2.000 mg, bevorzugt 500 mg bis 1.500 mg, besonders bevorzugt 800 mg bis 1.200 mg, bezogen auf eine Dosiereinheit der Kauzusammensetzung, insbesondere bezogen auf einen einzelnen Kaugummi, aufweisen.

Weiterhin ist es im Rahmen der vorliegenden Erfindung vorgesehen, daß die Kauzusammensetzung nach der Erfindung zusätzlich Vitalstoffe, insbesondere Vitamine, Mineralstoffe und/oder Spurenelemente, besonders bevorzugt aus der Gruppe von Resveratrol, Ascorbinsäure (Vitamin C), Vitamin B6 und/oder B12, Selen und Epicatechin, sowie deren Kombinationen, aufweist. Denn insbesondere Raucher mit einem regelmäßigen und starken Konsum an Rauchwaren weisen mitunter einen Mangel an den vorgenannten Vitalstoffen auf. Die gezielte Gabe von Vitalstoffen, insbesondere der vorgenannten Art, kann somit den bei Rauchern oftmals vorliegenden Mangel derartiger Substanzen kompensieren und damit das Wohlbefinden insgesamt verbessern. Vitalstoffe als solche können durch den menschlichen Körper weitgehend nicht selbst hergestellt und nur begrenzt gespeichert werden. Deshalb müssen dem Organismus diese Vitalstoffe regelmäßig und in ausreichenden Mengen zugeführt werden, was bei Rauchern aufgrund der obigen Ausführungen in besonderem Maße gilt.

In diesem Zusammenhang weist die erfindungsgemäße Zusammensetzung insbesondere mindestens ein Flavonoid auf. In diesem Zusammenhang handelt es sich bei dem Flavonoid um ein Phytoalexin, bevorzugt um Resveratrol. Resveratrol ist ein Phytoalexin mit starker antioxidativer Wirkung, welches pflanzlichen Ursprungs ist. Neben seinen antioxidativen Wirkungen kann Resveratrol den Fettstoffwechsel unterstützen, die Herz/Kreislauf-Funktion stärken und das Immunsystem aufgrund seiner antientzündlichen, antimutagenen und antikarzinogenen Wirkung unterstützen. Insbesondere bei Rauchern kann der Einsatz von antioxidativ wirkenden Substanzen vor dem Hintergrund durchgeführt werden, daß Raucher im allgemeinen einer höheren Belastung durch freie Radikale, welche insbesondere im Zigarettenrauch enthalten sind, ausgesetzt sind. Somit eignet sich Resveratrol insbesondere zur Verbesserung des Wohlbefindens bei Rauchern.

Im Rahmen der vorliegenden Erfindung kann das Phytoalexin, bevorzugt Resveratrol, in Mengen von 0,1 Gew.-% bis 10 Gew.-%, insbesondere 0,5 Gew.-% bis 7,5 Gew.-%, vorzugsweise 1 Gew.-% bis 5 Gew.-%, bevorzugt 1,5 Gew.-% bis 4 Gew.-%, bezogen auf die Kauzusammensetzung, eingesetzt werden. Mit anderen Worten kann die erfindungsgemäße Kauzusammensetzung das Phytoalexin, bevorzugt Resveratrol, in den vorgenannten Mengen aufweisen.

Die erfindungsgemäße Kauzusammensetzung kann das Phytoalexin, bevorzugt Resveratrol, in absoluten Mengen von 5 mg bis 400 mg, insbesondere 10 mg bis 300 mg, vorzugsweise 15 mg bis 200 mg, bevorzugt 20 mg bis 100 mg, besonders bevorzugt 30 mg bis 80 mg, bezogen auf eine Dosiereinheit der Kauzusammensetzung, insbesondere bezogen auf einen einzelnen Kaugummi, aufweisen. Weiterhin hat es sich als vorteilhaft erwiesen, wenn die erfindungsgemäße Kauzusammensetzung Ascorbinsäure (Vitamin C) aufweist. Dabei umfaßt der Begriff "Vitamin C", wie er im Rahmen der vorliegenden Erfindung verwendet wird, auch Derivate von Ascorbinsäure mit gleicher Wirkung, insbesondere alle Stoffe, die im Körper zur Ascorbinsäure umgesetzt werden können, wie beispielsweise Dehydroascorbinsäure. Ascorbinsäure stellt einen Radikalfänger dar und weist somit antioxidative Eigenschaften auf.

In diesem Zusammenhang weist die erfindungsgemäße Kauzusammensetzung die Ascorbinsäure (Vitamin C) in Mengen von 0,1 Gew.-% bis 10 Gew.-%, insbesondere 0,5 Gew.-% bis 7,5 Gew.-%, vorzugsweise 1 Gew.-% bis 5 Gew.-%, bevorzugt 1,5 Gew.-% bis 4 Gew.-%, bezogen auf die Kauzusammensetzung, auf.

Hinsichtlich der absoluten Mengen sollte die erfindungsgemäße Kauzusammensetzung die Ascorbinsäure (Vitamin C) in Mengen von 5 mg bis 400 mg, insbesondere 10 mg bis 300 mg, vorzugsweise 15 mg bis 200 mg, bevorzugt 20 mg bis 100 mg, besonders bevorzugt 30 mg bis 80 mg, bezogen auf eine Dosiereinheit der Kauzusammensetzung, insbesondere bezogen auf einen einzelnen Kaugummi, aufweisen.

Weiterhin weist die erfindungsgemäße Kauzusammensetzung ein Polyphenol, insbesondere mindestens ein Catechin, vorzugsweise Epicatechin, auf. Catechine stellen gleichermaßen Radikalfänger dar und weisen somit antioxidative Eigenschaften auf, wodurch sie sich insbesondere positiv bei Rauchern auswirken und einen Schutz vor Schäden durch freie Radikale, wie sie im Zigarettenrauch (z.B. Sauerstoffradikale) enthalten sind, bieten. Gleichermaßen weisen Catechine auch vorbeugende Wirkungen in bezug auf Herz/Kreislauf-Krankheiten auf. Zudem können Sie den Blutdruck und die Blutfette regulieren und bieten eine vorbeugende Wirkung vor Krebskrankheiten, so daß sie auch vor diesem Hintergrund bezüglich ihres Einsatzes bei Rauchern positiv zu beurteilen sind, da Raucher im allgemeinen ein erhöhtes Risiko aufweisen, an den vorgenannten Krankheiten zu erkranken.

Die erfindungsgemäß Kauzusammensetzung weist das Polyphenol, insbesondere Catechin, vorzugsweise Epicatechin, in Mengen von 0,2 Gew.-% bis 10 Gew.-%, insbesondere 0,3 Gew.-% bis 6 Gew.-%, vorzugsweise 0,5 Gew.-% bis 5 Gew.%, bevorzugt 1 Gew.-% bis 3 Gew.-%, bezogen auf die Kauzusammensetzung, auf. In absoluten Mengen kann die Kauzusammensetzung das Polyphenol, insbesondere Catechin, vorzugsweise Epicatechin, in Mengen von 1 mg bis 300 mg, insbesondere 5 mg bis 250 mg, vorzugsweise 10 mg bis 200 mg, bevorzugt 15 mg bis 100 mg, besonders bevorzugt 20 mg bis 50 mg, bezogen auf eine Dosiereinheit der Kauzusammensetzung, insbesondere bezogen auf einen einzelnen Kaugummi, aufweisen.

Darüber hinaus weist die erfindungsgemäße Kauzusammensetzung Selen auf . Hierbei handelt es sich um ein Spurenelement, welches sich auch aufgrund seiner gleichermaßen antioxidativen Eigenschaften für einen Einsatz bei Rauchern eignet.

In diesem Zusammenhang weist die erfindungsgemäße Kauzusammensetzung das Selen in Mengen von 1·10⁻⁷ Gew.-% bis 1·10⁻² Gew.-%, insbesondere 1·10⁻⁶ Gew.-% bis 1·10⁻³ Gew.-%, vorzugsweise 1·10⁻⁵ Gew.-% bis 1·10⁻³ Gew.-%, bevorzugt 0,5·10⁻⁴ Gew.-% bis 9,5·10⁻⁴ Gew.-%, bezogen auf die Kauzusammensetzung, auf.

In absoluten Mengen kann die erfindungsgemäß Kauzusammensetzung das Selen in Mengen von 0,01 µg bis 100 µg, insbesondere 0,1 µg bis 50 µg, vorzugsweise 0,5 µg bis 25 µg, bevorzugt 1 µg bis 20 µg, besonders bevorzugt 1 µg bis 10 µg, bezogen auf eine Dosiereinheit der Kauzusammensetzung, insbesondere bezogen auf einen einzelnen Kaugummi, aufweisen.

Neben den vorgenannten Vitalstoffen weist die erfindungsgemäße Kauzusammensetzung zudem mindestens ein Vitamin B, insbesondere Vitamin B6 und/oder Vitamin B12, auf. Die Bezeichnung "Vitamin B6", wie sie im Rahmen der vorliegenden Erfindung verwendet wird, stellt einen Sammelbegriff für 4,5-Bis(hydroxymethyl)-2-methylpyridin-3-ol (Pyridoxin) und dessen Derivate dar. Vitamin B6 entfaltet im allgemeinen seine Wirkungen im Aminosäurestoffwechsel. Raucher weisen oftmals einen Mangel an Vitamin B6 auf, so daß die zusätzliche Zufuhr von Vitamin B6 das Wohlempfinden bzw. die körperliche Verfassung eines Rauchers steigern kann, was auch der Raucherentwöhnung zugute kommt.

Gleichermaßen kann die erfindungsgemäße Kauzusammensetzung Vitamin B12 enthalten, welches ein Vertreter der Stoffgruppe der Cobalamine darstellt. Bei Vitamin B12 handelt es sich um die Verbindung 5-Desoxyadenosylcobalamin sowie um dessen Derivate mit gleicher Wirkung. Vitamin B12 nimmt beim Menschen an enzymatischen Reaktionen teil. Raucher weisen oftmals auch einen Mangel an Vitamin B12 auf, so daß eine zusätzliche Gabe von Vitamin B12 im Zusammenhang mit der Raucherentwöhnung gleichermaßen von Vorteil ist.

Was den Gehalt an den vorgenannten Vitaminen anbelangt, so weist die erfindungsgemäße Kauzusammensetzung das Vitamin B, insbesondere das Vitamin B6 und/oder das Vitamin B12, in Gesamtmengen von 0,5·10⁻⁷ Gew.-% bis 0,5·10⁻² Gew.-%, insbesondere 0,5·10⁻⁶ Gew.-% bis 0,5·10⁻³ Gew.-%, vorzugsweise 0,5·10⁻⁵ Gew.-% bis 5·10⁻⁴ Gew.-%, bevorzugt 1·10⁻⁵ Gew.-% bis 1·10⁻⁴ Gew.-%, bezogen auf die Kauzusammensetzung, auf.

Was die absolute Menge der vorgenannten Vitamine anbelangt, so kann die erfindungsgemäße Kauzusammensetzung das Vitamin B, insbesondere Vitamin B6 und/oder Vitamin B12, in Gesamtmengen von 0,005 µg bis 50 µg, insbesondere 0,01 µg bis 20 µg, vorzugsweise 0,05 µg bis 15 µg, bevorzugt 0,1 µg bis 10 µg, besonders bevorzugt 0,5 µg bis 5 µg, bezogen auf eine Dosiereinheit der Kauzusammensetzung, insbesondere bezogen auf einen einzelnen Kaugummi, aufweisen.

Durch die gezielte Ausstattung der erfindungsgemäßen Kauzusammensetzung mit den vorgenannten Vitalstoffen, insbesondere Resveratrol, Ascorbinsäure (Vitamin C), Vitamin B6 und/oder Vitamin B12, Selen, und Epicatechin sowie deren Kombinationen, können bei Rauchern oftmals auftretende Mangelzustände verbessert und die Belastung durch freie Radikale verringert werden, so daß es insgesamt zu einer Steigerung des Wohlbefindens kommt, was auch zu einer erleichterten Raucherentwöhnung führt, da sich der zu entwöhnende Raucher in einer besseren Verfassung befindet und sich dadurch bedingt auch besser fühlt.

Darüber hinaus kann die erfindungsgemäße Kauzusammensetzung außerdem mindestens einen weiteren Wirk- bzw. Inhaltsstoff enthalten, welcher eine pharmakologische Wirkung aufweisen kann, welche die Raucherentwöhnung positiv beeinflussen kann. Der Fachmann ist jederzeit in der Lage, sowohl die konkreten Substanzen als auch die diesbezüglich zu wählenden Mengen in bezug auf die erfindungsgemäße Kauzusammensetzung auszuwählen bzw. anzupassen.

Schließlich kann die Kauzusammensetzung nach der Erfindung außerdem mindestens einen weiteren Inhaltsstoff, ausgewählt aus der Gruppe von Zusatz- und/oder Hilfsstoffen, wie Füll-, Streck-, Binde-, Netz-, Stabilisierungs-, Färbe-, Puffer-, Riech-, Geschmacks-, Süßungs-, Aroma-, Verarbeitungshilfs- und Konservierungsstoffen und -mitteln sowie deren Mischungen enthalten. Der Fachmann ist jederzeit in der Lage, sowohl die konkreten Substanzen als auch die diesbezüglich zu wählenden Mengen in bezug auf die erfindungsgemäße Kauzusammensetzung auszuwählen bzw. anzupassen. Als Süßungsmittel kann beispielsweise Sucralose eingesetzt werden.

Gemäß einer erfindungsgemäß besonders bevorzugten Ausführungsform enthält die erfindungsgemäße Kauzusammensetzung, vorzugsweise der Kaugummi, insbesondere zu Raucherentwöhnung,
a) eine Kaugummigrundmasse, in Mengen von 20 Gew.-% bis 95 Gew.-%, insbesondere 30 Gew.-% bis 90 Gew.-%, vorzugsweise 40 Gew.-% bis 80 Gew.-%, bevorzugt 50 Gew.-% bis 70 Gew.-%,
b) einen Kautabak, in Mengen von 5 Gew.-% bis 60 Gew.-%, insbesondere 10 Gew.-% bis 50 Gew.-%, vorzugsweise 20 Gew.-% bis 40 Gew.-%, bevorzugt 25 Gew.-% bis 35 Gew.-%,
c) mindestens ein Phytoalexin, bevorzugt Resveratrol, in Mengen von 0,1 Gew.-% bis 10 Gew.%, insbesondere 0,5 Gew.-% bis 7,5 Gew.-%, vorzugsweise 1 Gew.-% bis 5 Gew.-%, bevorzugt 1,5 Gew.-% bis 4 Gew.-%,
d) Ascorbinsäure (Vitamin C), in Mengen von 0,1 Gew.-% bis 10 Gew.-%, insbesondere 0,5 Gew.-% bis 7,5 Gew.-%, vorzugsweise 1 Gew.-% bis 5 Gew.-%, bevorzugt 1,5 Gew.-% bis 4 Gew.-%,
e) mindestens ein Polyphenol, insbesondere mindestens ein Catechin, vorzugsweise Epicatechin, in Mengen von 0,2 Gew.-% bis 10 Gew.-%, insbesondere 0,3 Gew.-% bis 6 Gew.-%, vorzugsweise 0,5 Gew.-% bis 5 Gew.-%, bevorzugt 1 Gew.-% bis 3 Gew.-%,
f) Selen, in Mengen von 1·10⁻⁷ Gew.-% bis 1·10² Gew.-%, insbesondere 1·10⁻⁶ Gew.-% bis 1·10⁻³ Gew.-%, vorzugsweise 1·10⁻⁵ Gew.-% bis 1·10⁻³ Gew.-%, bevorzugt 0,5·10⁻⁴ Gew.-% bis 9,5·10⁻⁴ Gew.-%,
g) mindestens ein Vitamin B, insbesondere Vitamin B6 und/oder Vitamin B12, in Gesamtmengen von 0,5·10⁻⁷ Gew.-% bis 0,5·10⁻² Gew.-%, insbesondere 0,5·10⁻⁶ Gew.-% bis 0,5·10⁻³ Gew.-%, vorzugsweise 0,5·10⁻⁵ Gew.-% bis 5·10⁻⁴ Gew.-%, bevorzugt 1·10⁻⁵ Gew.-% bis 1·10⁻⁴ Gew.-%,
wobei die Mengenangaben jeweils auf die Kauzusammensetzung bezogen sind.

Gemäß einer weiteren besonders bevorzugten erfindungsgemäßen Ausführungsform enthält die erfindungsgemäße Kauzusammensetzung, vorzugsweise der Kaugummi, insbesondere zur Raucherentwöhnung,
a) eine Kaugummigrundmasse, insbesondere in Mengen von 100 mg bis 5.000 mg, insbesondere 200 mg bis 3.000 mg, vorzugsweise 300 mg bis 2.000 mg, bevorzugt 500 mg bis 1.500 mg, besonders bevorzugt 800 mg bis 1.200 mg,
b) einen Kautabak, insbesondere in Mengen von 50 mg bis 1.000 mg, insbesondere 200 mg bis 800 mg, vorzugsweise 300 mg bis 700 mg, bevorzugt 400 mg bis 600 mg,
c) mindestens ein Phytoalexin, bevorzugt Resveratrol, insbesondere in Mengen von 5 mg bis 400 mg, insbesondere 10 mg bis 300 mg, vorzugsweise 15 mg bis 200 mg, bevorzugt 20 mg bis 100 mg, besonders bevorzugt 30 mg bis 80 mg,
d) Ascorbinsäure (Vitamin C), insbesondere in Mengen von 5 mg bis 400 mg, insbesondere 10 mg bis 300 mg, vorzugsweise 15 mg bis 200 mg, bevorzugt 20 mg bis 100 mg, besonders bevorzugt 30 mg bis 80 mg,
e) mindestens ein Polyphenol, insbesondere mindestens ein Catechin, vorzugsweise Epicatechin, insbesondere in Mengen von 1 mg bis 300 mg, insbesondere 5 mg bis 250 mg, vorzugsweise 10 mg bis 200 mg, bevorzugt 15 mg bis 100 mg, besonders bevorzugt 20 mg bis 50 mg,
f) Selen, insbesondere in Mengen von 0,01 µg bis 100 µg, insbesondere 0,1 µg bis 50 µg, vorzugsweise 0,5 µg bis 25 µg, bevorzugt 1 µg bis 20 µg, besonders bevorzugt 1 µg bis 10 µg,
g) mindestens ein Vitamin B, insbesondere Vitamin B6 und/oder Vitamin B12, insbesondere in Gesamtmengen von 0,005 µg bis 50 µg, insbesondere 0,01 µg bis 20 µg, vorzugsweise 0,05 µg bis 15 µg, bevorzugt 0,1 µg bis 10 µg, besonders bevorzugt 0,5 µg bis 5 µg,
wobei die Mengenangaben jeweils auf eine Dosiereinheit der Kauzusammensetzung, insbesondere auf einen einzelnen Kaugummi, bezogen sind.

Was die erfindungsgemäße Kauzusammensetzung schließlich anbelangt, so kann diese - wie zuvor angeführt - in Form einer Dosiereinheit, insbesondere als Kaugummi, vorliegen. In diesem Zusammenhang sollte die Dosiereinheit, insbesondere der Kaugummi, eine Masse bzw. ein Gewicht von 100 mg bis 10 g, insbesondere 200 mg bis 5 g, vorzugsweise 500 mg bis 3 g, bevorzugt 1 g bis 2 g, aufweisen.

Weiterhin kann es im Rahmen der vorliegenden Erfindung vorgesehen sein, dass eine Verpackungseinheit mindestens eine, insbesondere eine Vielzahl von Dosiereinheiten der erfindungsgemäßen Kauzusammensetzung, wie zuvor definiert, vorzugsweise eine Vielzahl einzelner Kaugummis, aufweist. Die Verpackungseinheit kann insbesondere in Form eines Behältnisses, einer Umverpackung, einer Dose, einer Sichtverpackung, eines Blisters oder dergleichen vorliegen. In diesem Zusammenhang können die erfindungsgemäßen Kauzusammensetzungen auf Basis der einzelnen Dosiereinheiten, insbesondere in Form von Kaugummis, beispielsweise zur Einzelentnahme separat verpackt sein. Gleichermaßen kann die Verpackungseinheit auch in Form eines Vorratsbehältnisses zur Aufnahme einer größeren Menge der erfindungsgemäßen Kauzusammensetzung in Form von Dosiereinheiten, insbesondere Kaugummis, ausgebildet sein, wobei die Dosiereinheiten nicht voneinander getrennt aufbewahrt werden. In diesem Fall kann die erfindungsgemäße Verpackungseinheit beispielsweise mit einer vorzugsweise wiederverschließbaren Verschlußvorrichtung oder mit einem Entnahme- bzw. Abgabesystem ausgestattet sein ("Spender").

Die erfindungsgemäße Kauzusammensetzung, insbesondere wie zuvor definiert, kann im Rahmen der vorliegenden Erfindung insbesondere zur Raucherentwöhnung und/oder zur Erleichterung der Aufgabe des Rauchens und/oder zur Behandlung von Tabak- und Nikotinabhängigkeit und/oder zur Linderung von Nikotinentzugssymptomen verwendet werden.

In diesem Zusammenhang kann die erfindungsgemäße Kauzusammensetzung beispielsweise zur Verabreichung einer Gesamttagesdosis von 2 mg bis 80 mg pro Tag bei einer Verabreichung von 1 bis 20 Dosiereinheiten, insbesondere in Form einzelner Kaugummis, hergerichtet sein. Diesbezüglich sollte die Gesamttagesdosis bzw. die Anzahl der zu verabreichenden Dosiereinheiten bzw. Kaugummis in Abhängigkeit von der schwere der Rauchwarenabhängigkeit ausgewählt werden.

In diesem Zusammenhang kann insbesondere zu Beginn des Rauchverzichtes eine höhere Menge an erfindungsgemäßen Dosiereinheiten mit gegebenenfalls höherer Nikotinkonzentration bzw. -menge pro Dosiereinheit verabreicht werden. So können beispielsweise pro Tag zu Beginn der Raucherentwöhnung beispielsweise etwa zehn bis fünfzehn oder mehr erfindungsgemäße Kauzusammensetzungen in Form von Dosiereinheiten, insbesondere Kaugummis, mit einem Nikotingehalt von beispielsweise 4 mg oder 6 mg oder mehr pro Dosiereinheit über den Tag verteilt gekaut werden. Dabei sollte die erfindungsgemäße Kauzusammensetzung vorzugsweise langsam und/oder über einen Zeitraum von mindestens 10 min, insbesondere mindestens 15 min, vorzugsweise mindestens 30 min oder mehr, gekaut werden, damit die kontrollierte und zeitliche gesteuerte Freisetzung des Nikotins noch zusätzlich unterstützt wird. Durch das Kauen wird das Nikotin im zeitlichen Verlauf aus der erfindungsgemäßen Kauzusammensetzung freigesetzt. Das Nikotin wird dabei in den Körper vorwiegend über die Mundschleimhaut sowie durch Verschlucken aufgenommen. Auf diese Weise entsteht im Blutplasma eine über einen längeren Zeitraum erhöhte Nikotinkonzentration, die das Rauchverlangen über einen längeren Zeitraum vermindert bzw. unterbindet.

Im weiteren Verlauf der Raucherentwöhnung kann die Nikotindosis allmählich verringert werden. So sollte - je nach Bedürfnis des zu entwöhnenden Rauchers - beispielsweise nach etwa zwei Wochen eine geringere Stückzahl an Dosiereinheiten der erfindungsgemäßen Kauzusammensetzung, insbesondere in Form von Kaugummis, gekaut werden, beispielsweise etwa sechs bis zehn Stück pro Tag mit einer Nikotinmenge von beispielsweise 2 mg je Kaugummi. Im weiteren zeitlichen Verlauf mit zunehmender Entwöhnung kann die Dosierung noch weiter verringert werden, wobei zum Ende der Raucherentwöhnung beispielsweise nur noch eine Dosiereinheit der erfindungsgemäßen Kauzusammensetzung, insbesondere in Form eines Kaugummis, pro Tag gekaut werden sollte. Anschließend kann die erfindungsgemäße Kauzusammensetzung sogar vollständig abgesetzt werden, ohne daß das Rauchverlangen wieder auflebt.

Insgesamt wird im Rahmen der vorliegenden Erfindung somit eine gut wirksame Kauzusammensetzung mit optimierter Wirkstofffreisetzung und guten organoleptischen Eigenschaften bereitgestellt.

Es versteht sich von selbst, daß Ausgestaltungen, Ausführungsformen, Vorteile und dergleichen, welche nachfolgend zu Zwecken der Vermeidung von Wiederholungen nur zu einem Erfindungsaspekt ausgeführt sind, selbstverständlich auch in bezug auf die übrigen Erfindungsaspekte entsprechend gelten.

### Ausführungsbeispiele:

### 1. Zusammensetzung A (erfindungsgemäß):

In an sich bekannter Weise wurde eine erfindungsgemäße Kauzusammensetzung hergestellt, wobei in diesem Zusammenhang ein handelsüblicher Kautabak eingesetzt wurde, welcher zerkleinert bzw. vermahlen und gleichmäßig in eine handelsübliche Kaugummigrundmasse eingearbeitet wurde, so daß eine homogene Verteilung des Kautabaks in der Kaugummigrundmasse resultierte. Nachfolgend wurden die übrigen Inhaltsstoffe, wie in der nachfolgenden Tabelle spezifiziert, in die erfindungsgemäße Kauzusammensetzung eingearbeitet. Die nachfolgende Tabelle zeigt die Zusammensetzung der erfindungsgemäßen Kauzusammensetzung:

| **Inhaltsstoff** | **Menge in Gewichtsteilen** |
|---|---|
| Kaugummigrundmasse | 1.000 |
| Kautabak (nitrosaminfrei) | 500 |
| Resveratrol | 50 |
| Ascorbinsäure | 50 |
| Epicatechin | 30 |
| Selen | 0,004 |
| Vitamin B6 | 0,0005 |
| Vitamin B12 | 0,001 |
| | |
| Additive (Aromen, Verarbeitungshilfsmittel, Süßungsmittel, Füllstoffe) | ad 1.700 |

Die auf diese Weise hergestellte Zusammensetzung wurde nachfolgend zu einzelnen erfindungsgemäßen Kaugummis zur Raucherentwöhnung (1,7 g Zusammensetzung pro Kaugummi) verarbeit, welche angenehm im Geschmack und insgesamt gut formbar sind. Die Kaugummis können gegebenenfalls noch in an sich bekannter Weise mit einer Beschichtung versehen werden.

### 2. Bestimmung der Blutplasmakonzentration von Nikotin bei Verabreichung der erfindungsgemäßen Kauzusammensetzung im Vergleich zu Applikationsformen des Standes der Technik:

Im Rahmen der vorliegenden Untersuchungen wurde der zeitliche Verlauf der Nikotinkonzentration im Blutplasma bei jeweils vier Probanden für die erfindungsgemäße Kauzusammensetzung sowie für Verabreichungen des Standes der Technik bestimmt.

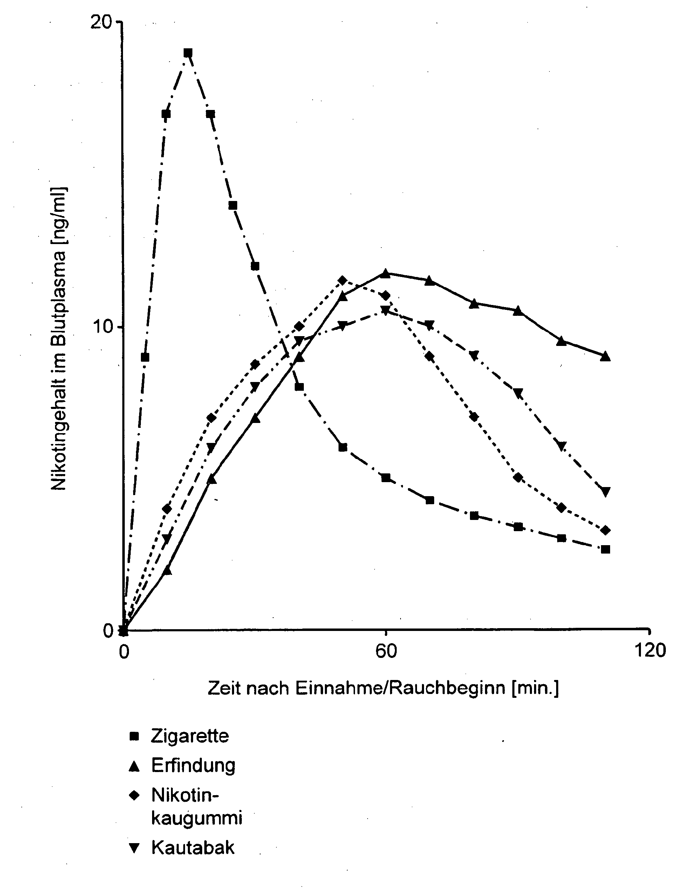

Die in der einzigen Figur dargestellten Werte stellen die jeweiligen Mittelwerte auf Basis der für die vier Probanden ermittelten Einzelwerte dar. Der Zeitpunkt t = 0 stellt den Beginn der Nikotinverabreichung dar. Hierzu wurde neben der erfindungsgemäßen Kauzusammensetzung (vgl. "Erfindung") eine handelsübliche Filterzigarette (vgl. "Zigarette") und ein handelsübliches Nikotinkaugummi mit Nikotin in Reinform (vgl. "Nikotinkaugummi") verwendet, welches eine zu der erfindungsgemäßen Kauzusammensetzung identische Nikotinmenge aufwies. Schließlich wurde als weiterer Vergleich ein handelsüblicher Kautabak (vgl. "Kautabak") mit zur erfindungsgemäßen Kauzusammensetzung vergleichbarer Nikotinmenge verabreicht.

Die einzige Figur veranschaulicht den jeweiligen zeitlichen Verlauf der Nikotinkonzentration im Blutplasma nach Verabreichung der jeweiligen nikotinhaltigen Applikationsform, wobei die Zigarette ("Zigarette") für etwa sechs Minuten geraucht wurde (bis t = 6 min); die jeweiligen Kauzusammensetzungen dagegen ("Erfindung", "Nikotinkaugummi", "Kautabak") wurden für jeweils etwa 90 min gekaut.

Die einzige Figur zeigt, daß bei dem Versuch "Zigarette" die maximale Konzentration von Nikotin im Blutplasma in Form eines sehr hohen Spitzenwertes bereits nach etwa 15 min erreicht wird, danach fällt die Nikotinkonzentration bei der Zigarette deutlich ab. Die Kauzusammensetzungen ("Erfindung", "Nikotinkaugummi", "Kautabak") weisen im Gegensatz dazu einen Verlauf ohne einen schnellen Anstieg auf einen kurzzeitigen Spitzenwert auf.

Im Vergleich zu dem handelsüblichen Nikotinkaugummi mit reinem Nikotin und dem handelsüblichen Kautabak ("Nikotinkaugummi", "Kautabak") weist die erfindungsgemäße Zusammensetzung ("Erfindung") zudem eine deutlich verzögerte bzw. retardierte Anfangsfreisetzung auf. Im weiteren grundlegenden Unterschied zu den Verabreichungsformen des Standes der Technik ("Nikotinkaugummi", "Kautabak") zeichnet sich die erfindungsgemäße Zusammensetzung ("Erfindung") zudem dadurch aus, daß die Nikotinkonzentration im Blutplasma im weiteren zeitlichen Verlauf deutlich weniger stark abfällt, als dies bei den Vergleichzusammensetzungen ("Nikotinkaugummi", "Kautabak") der Fall ist. Mit Hilfe der erfindungsgemäßen Kauzusammensetzung wird somit gewährleistet, daß die Nikotinkonzentration im Blutplasma auch für längere Zeiträume nach Verabreichungsbeginn relativ hohe Werte aufweist.

Die vorliegenden Untersuchungsergebnisse zeigen somit die hervorragende kontrollierte bzw. retardierte Freisetzung von Nikotin aus der erfindungsgemäßen Kauzusammensetzung, so daß auch bei größeren Zeitdauern nach Beginn des Kauens noch relativ hohe Nikotinkonzentrationen im Blutplasma vorliegen.

### 3. Anwendungsbeobachtungen bzw. Wirksamkeitsstudien:

a) Im Rahmen einer Studie zur Raucherentwöhnung wurden insgesamt 15 Probanden, welche jeweils zigarettenabhängig waren und jeweils vor Versuchsbeginn mehr als 20 Zigaretten pro Tag konsumierten, mit der erfindungsgemäßen Kauzusammensetzung gemäß Zusammensetzung A versorgt, wobei in den ersten zwei Wochen täglich jeweils vier bis sechs Kaugummis gemäß Zusammensetzung A gekaut wurden. Nach 14 Tagen wurde die Anzahl an Dosiereinheiten halbiert und nach weiteren 14 Tagen - also einen Monat nach Beginn der Verabreichung - wurde nur noch eine erfindungsgemäße Dosiereinheit bzw. eine erfindungsgemäße Kauzusammensetzung pro Tag gekaut. Nach weiteren 14 Tagen wurde die Verabreichung vollständig eingestellt. Während der gesamten Versuchsdauer wurden keine Zigaretten geraucht. Die Probanden beurteilten die erfindungsgemäße Kauzusammensetzung auf Basis eines Kaugummis in bezug auf die organoleptischen Eigenschaften positiv, wobei insbesondere von einem angenehmen Kaugefühl bei gutem Geschmack und guter Verträglichkeit berichtet wurde. Zudem hatte bereits eine überwiegende Mehrheit der Pobanden bereits nach kurzer Zeit ein stark verringertes Rauchbedürfnis. Drei Monate nach Beendigung der Verabreichung der erfindungsgemäßen Kauzusammensetzung rauchten vier Probanden erneut. Bei den übrigen elf Probanden kam es zu keinen Rückfallerscheinungen.
b) In einer weiteren Wirksamkeitsstudie wurde insgesamt weiteren 15 Probanden, welche vor Aufnahme der Verabreichung jeweils mehr als 20 Zigaretten pro Tag rauchten, ein handelsübliches Nikotinkaugummi gemäß dem unter a) beschriebenen Schema verabreicht, wobei das handelsübliche Nikotinkaugummi eine zu der erfindungsgemäßen Zusammensetzung vergleichbare Menge an Nikotin aufwies. Während der Studiendauer klagten drei Probanden über Magenprobleme, und ein Teil der Probanden stellten das schlechte "Kaugefühl" der Zusammensetzung des Standes der Technik heraus. Drei Monate nach Beendigung der Verabreichung der Nikotinkaugummis des Standes der Technik rauchten insgesamt sieben Probanden erneut, bei den übrigen acht Probanden traten keine Rückfallerscheinungen auf.
c) In einer weiteren Wirksamkeitsstudie erhielten weitere 15 Probanden, welche vor Beginn der Untersuchungen jeweils mehr als 20 Zigaretten pro Tag rauchten, zur Raucherentwöhnung Nikotinpflaster des Standes der Technik, wobei während der ersten zwei Wochen einmal täglich ein Nikotinpflaster mit etwa 25 mg Nikotin auf eine Hautstelle aufgeklebt wurde. In den nachfolgenden zwei Wochen wurde jeweils einmal täglich ein Nikotinpflaster mit etwa 15 mg Nikotin pro Pflaster verabreicht. Für die letzten zwei Wochen wurde jeweils ein Nikotinpflaster täglich mit einer Nikotinmenge von etwa 10 mg Nikotin pro Pflaster verabreicht. Bei zwei Probanden zeigten sich leichte bis mittlere Hautirritationen an der Pflasterstelle. Die Probanden beklagten sich mehrheitlich während der Verabreichungsdauer über ein mittel bis stark ausgeprägtes Rauchbedürfnis, was insbesondere auch auf das Fehlen einer,motorischen Ersatzhandlung für das Rauchen während der Verabreichungsdauer zurückgeführt worden ist. Drei Monate nach Beendigung der Pflasterapplikation rauchten zehn Patienten erneut. Fünf Patienten zeigten keine Rückfallerscheinungen.

Die zuvor beschriebenen Anwendungs- bzw. Wirksamkeitsstudien belegen insgesamt die hervorragende Wirksamkeit bzw. Effektivität der erfindungsgemäßen Kauzusammensetzung gegenüber dem Stand der Technik, wobei sowohl ein positives Kau- bzw. Geschmacksgefühl mit der erfindungsgemäßen Zusammensetzung in Verbindung gebracht wird als auch insgesamt niedrigere Rückfallquoten nach Ende der Verabreichung der erfindungsgemäßen Kauzusammensetzung auftreten.

## Patentansprüche

1. Kauzusammensetzung in Form eines Kaugummis, insbesondere zur Raucherentwöhnung, wobei die Kauzusammensetzung neben
a) 20 Gew.-% bis 95 Gew.-% einer Kaugummigrundmasse
b) 5 Gew.-% bis 60 Gew.-% eines nikotinhaltigen Kautabaks
enthält, wobei die Kauzusammensetzung zudem
c) mindestens ein Phytoalexin in Mengen von 0,1 Gew.-% bis 10 Gew.-%,
d) Ascorbinsäure (Vitamin C) in Mengen von 0,1 Gew.-% bis 10 Gew.-%,
e) mindestens ein Polyphenol in Mengen von 0,2 Gew.-% bis 10 Gew.-%,
f) Selen in Mengen von 1·10⁻⁷ Gew.-% bis 1·10⁻² Gew.-% und
g) mindestens ein Vitamin B in Mengen von 0,5·10⁻⁷ Gew.-% bis 0,5·10⁻² Gew.-%
enthält, wobei die Mengenangaben jeweils auf die Kauzusammensetzung bezogen sind.

2. Kauzusammensetzung nach Anspruch 1,
wobei der Kautabak in die Kaugummigrundmasse eingearbeitet und/oder eingebettet ist und/oder
wobei der Kautabak in zumindest im wesentlichen homogener Verteilung in die Kaugummigrundmasse eingearbeitet und/oder eingebettet ist und/oder
wobei der Kautabak in zerkleinerter und/oder feinverteilter Form, insbesondere pulverisiert oder vermahlen, in die Kaugummigrundmasse eingearbeitet und/oder eingebettet ist.

3. Kauzusammensetzung nach Anspruch 1 oder 2, wobei der Kautabak eine mittlere Partikel- und/oder Fasergröße von 0,001 µm bis 5 mm, insbesondere 0,01 µm bis 3 mm, vorzugsweise 0,1 µm bis 2 mm, bevorzugt 1 µm bis 1 mm, aufweist.

4. Kauzusammensetzung nach einem der vorangehenden Ansprüche,
wobei der Kautabak einen Nikotingehalt von 0,01 Gew.-% bis 2 Gew.-%, insbesondere 0,05 Gew.-% bis 1,5 Gew.-%, vorzugsweise 0,1 Gew.-% bis 1,25 Gew.-%, bevorzugt 0,2 Gew.-% bis 1 Gew.-%, bezogen auf den Kautabak, aufweist und/oder
wobei die Kauzusammensetzung eine Nikotinmenge von 0,1 mg bis 20 mg, insbesondere 0,5 mg bis 15 mg, vorzugsweise 0,75 mg bis 10 mg, bevorzugt 1 mg bis 5 mg, bezogen auf eine Dosiereinheit der Kauzusammensetzung, insbesondere bezogen auf einen einzelnen Kaugummi, aufweist.

5. Kauzusammensetzung nach einem der vorangehenden Ansprüche,
wobei der Kautabak einen Nitrosamingehalt von höchstens 5 µg Nitrosamin(e) / g Kautabak, insbesondere höchstens 2 µg Nitrosamin(e) / g Kautabak, vorzugsweise höchstens 1 µg Nitrosamin(e) / g Kautabak, bevorzugt höchstens 0,5 µg Nitrosamin(e) / g Kautabak, besonders bevorzugt höchstens 1 µg Nitrosamin(e) / g Kautabak, aufweist und ganz besonders bevorzugt zumindest im wesentlichen nitrosaminfrei ausgebildet ist.

6. Kauzusammensetzung nach einem der vorangehenden Ansprüche,
wobei die Kauzusammensetzung den Kautabak in Mengen von 10 Gew.-% bis 50 Gew.-%, vorzugsweise 20 Gew.-% bis 40 Gew.-%, bevorzugt 25 Gew.-% bis 35 Gew.-%, bezogen auf die Kauzusammensetzung, aufweist und/oder
wobei die Kauzusammensetzung den Kautabak in Mengen von 50 mg bis 1.000 mg, insbesondere 200 mg bis 800 mg, vorzugsweise 300 mg bis 700 mg, bevorzugt 400 mg bis 600 mg, bezogen auf eine Dosiereinheit der Kauzusammensetzung, insbesondere bezogen auf einen einzelnen Kaugummi, aufweist.

7. Kauzusammensetzung nach einem der vorangehenden Ansprüche,
wobei die Kaugummigrundmasse zumindest im wesentlichen wasserunlöslich, insbesondere in Speichel zumindest im wesentlichen unlöslich, und/oder zumindest im wesentlichen unverdaulich ist und/oder
wobei die Kaugummigrundmasse natürliche Polymere, insbesondere Kautschuk, vorzugsweise in Form von Latex, Crepe oder Sheets, und/oder Polyole, enthält und/oder wobei die Kaugummigrundmasse synthetische Polymere, insbesondere synthetische Thermoplaste, vorzugsweise Polyvinylether, insbesondere Poly(ethylvinylether) und/oder Poly(isobutylvinylether), und/oder Polyisobutene, enthält.

8. Kauzusammensetzung nach einem der vorangehenden Ansprüche,
wobei die Kauzusammensetzung die Kaugummigrundmasse in Mengen von 30 Gew.-% bis 90 Gew.-%, vorzugsweise 40 Gew.-% bis 80 Gew.-%, bevorzugt 50 Gew.-% bis 70 Gew.-%, bezogen auf die Kauzusammensetzung, aufweist und/oder
wobei die Kauzusammensetzung die Kaugummigrundmasse in Mengen von 100 mg bis 5.000 mg, insbesondere 200 mg bis 3.000 mg, vorzugsweise 300 mg bis 2.000 mg, bevorzugt 500 mg bis 1.500 mg, besonders bevorzugt 800 mg bis 1.200 mg, bezogen auf eine Dosiereinheit der Kauzusammensetzung, insbesondere bezogen auf einen einzelnen Kaugummi, aufweist.

9. Kauzusammensetzung nach einem der vorangehenden Ansprüche,
wobei die Kauzusammensetzung das Phytoalexin, bevorzugt Resveratrol, in Mengen von 0,5 Gew.-% bis 7,5 Gew.-%, vorzugsweise 1 Gew.-% bis 5 Gew.-%, bevorzugt 1,5 Gew.-% bis 4 Gew.-%, bezogen auf die Kauzusammensetzung, aufweist und/oder
wobei die Kauzusammensetzung das Phytoalexin, bevorzugt Resveratrol, in Mengen von 5 mg bis 400 mg, insbesondere 10 mg bis 300 mg, vorzugsweise 15 mg bis 200 mg, bevorzugt 20 mg bis 100 mg, besonders bevorzugt 30 mg bis 80 mg, bezogen auf eine Dosiereinheit der Kauzusammensetzung, insbesondere bezogen auf einen einzelnen Kaugummi, aufweist.

10. Kauzusammensetzung nach einem der vorangehenden Ansprüche,
wobei die Kauzusammensetzung die Ascorbinsäure (Vitamin C) in Mengen von 0,5 Gew.-% bis 7,5 Gew.-%, vorzugsweise 1 Gew.-% bis 5 Gew.-%, bevorzugt 1,5 Gew.-% bis 4 Gew.-%, bezogen auf die Kauzusammensetzung, aufweist und/oder
wobei die Kauzusammensetzung die Ascorbinsäure (Vitamin C) in Mengen von 5 mg bis 400 mg, insbesondere 10 mg bis 300 mg, vorzugsweise 15 mg bis 200 mg, bevorzugt 20 mg bis 100 mg, besonders bevorzugt 30 mg bis 80 mg, bezogen auf eine Dosiereinheit der Kauzusammensetzung, insbesondere bezogen auf einen einzelnen Kaugummi, aufweist.

11. Kauzusammensetzung nach einem der vorangehenden Ansprüche,
wobei die Kauzusammensetzung das Polyphenol, insbesondere Catechin, vorzugsweise Epicatechin, in Mengen von 0,3 Gew.-% bis 6 Gew.-%, vorzugsweise 0,5 Gew.-% bis 5 Gew.-%, bevorzugt 1 Gew.-% bis 3 Gew.-%, bezogen auf die Kauzusammensetzung, aufweist und/oder
wobei die Kauzusammensetzung das Polyphenol, insbesondere Catechin, vorzugsweise Epicatechin, in Mengen von 1 mg bis 300 mg, insbesondere 5 mg bis 250 mg, vorzugsweise 10 mg bis 200 mg, bevorzugt 15 mg bis 100 mg, besonders bevorzugt 20 mg bis 50 mg, bezogen auf eine Dosiereinheit der Kauzusammensetzung, insbesondere bezogen auf einen einzelnen Kaugummi, aufweist.

12. Kauzusammensetzung nach einem der vorangehenden Ansprüche,
wobei die Kauzusammensetzung das Selen in Mengen von 1·10⁻⁶ Gew.-% bis 1·10⁻³ Gew.-%, vorzugsweise 1·10⁻⁵ Gew.-% bis 1·10⁻³ Gew.-%, bevorzugt 0,5·10⁻⁴ Gew.-% bis 9,5·10⁻⁴ Gew.-%, bezogen auf die Kauzusammensetzung, aufweist und/oder
wobei die Kauzusammensetzung das Selen in Mengen von 0,01 µg bis 100 µg, insbesondere 0,1 µg bis 50 µg, vorzugsweise 0,5 µg bis 25 µg, bevorzugt 1 µg bis 20 µg, besonders bevorzugt 1 µg bis 10 µg, bezogen auf eine Dosiereinheit der Kauzusammensetzung, insbesondere bezogen auf einen einzelnen Kaugummi, aufweist.

13. Kauzusammensetzung nach einem der vorangehenden Ansprüche,
wobei die Kauzusammensetzung das Vitamin B, insbesondere Vitamin B6 und/oder Vitamin B12, in Gesamtmengen von 0,5·10⁻⁶ Gew.-% bis 0,5·10⁻³ Gew.-%, vorzugsweise 0,5·10⁻⁵ Gew.-% bis 5·10⁻⁴ Gew.-%, bevorzugt 1·10⁻⁵ Gew.-% bis 1·10⁻⁴ Gew.-%, bezogen auf die Kauzusammensetzung, aufweist und/oder
wobei die Kauzusammensetzung das Vitamin B, insbesondere Vitamin B6 und/oder Vitamin B12, in Gesamtmengen von 0,005 µg bis 50 µg, insbesondere 0,01 µg bis 20 µg. vorzugsweise 0,05 µg bis 15 µg, bevorzugt 0,1 µg bis 10 µg, besonders bevorzugt 0,5 µg bis 5 µg, bezogen auf eine Dosiereinheit der Kauzusammensetzung, insbesondere bezogen auf einen einzelnen Kaugummi, aufweist.

14. Kauzusammensetzung nach einem der vorangehenden Ansprüche, enthaltend außerdem mindestens einen weiteren Inhaltsstoff, ausgewählt aus der Gruppe von Zusatz- und/oder Hilfsstoffen, wie Füll-, Streck-, Binde-, Netz-, Stabilisierungs-, Färbe-, Puffer-, Riech-, Geschmacks-, Süßungs-, Aroma-, Verarbeitungshilfs- und Konservierungsstoffen und -mitteln sowie deren Mischungen.

15. Kauzusammensetzung in Form eines Kaugummis nach einem der vorangehenden Ansprüche, insbesondere zur Raucherentwöhnung, enthaltend
a) eine Kaugummigrundmasse in Mengen von 30 Gew.-% bis 90 Gew.-%, vorzugsweise 40 Gew.-% bis 80 Gew.-%, bevorzugt 50 Gew.-% bis 70 Gew.-%,
b) einen Kautabak in Mengen von 10 Gew.-% bis 50 Gew.-%, vorzugsweise 20 Gew.-% bis 40 Gew.-%, bevorzugt 25 Gew.-% bis 35 Gew.-%,
c) mindestens ein Phytoalexin, bevorzugt Resveratrol, in Mengen von 0,5 Gew.-% bis 7,5 Gew.-%, vorzugsweise 1 Gew.-% bis 5 Gew.-%, bevorzugt 1,5 Gew.-% bis 4 Gew.-%,
d) Ascorbinsäure (Vitamin C) in Mengen von 0,5 Gew.-% bis 7,5 Gew.-%, vorzugsweise 1 Gew.-% bis 5 Gew.-%, bevorzugt 1,5 Gew.-% bis 4 Gew.-%,
e) mindestens ein Polyphenol, insbesondere mindestens ein Catechin, vorzugsweise Epicatechin, in Mengen von 0,3 Gew.-% bis 6 Gew.-%, vorzugsweise 0,5 Gew.-% bis 5 Gew.-%, bevorzugt 1 Gew.-% bis 3 Gew.-%,
f) Selen in Mengen von 1·10⁻⁷ Gew.-% bis 1·10⁻² Gew.-%, insbesondere 1·10⁻⁶ Gew.-% bis 1·10⁻³ Gew.-%, vorzugsweise 1·10⁻⁵ Gew.-% bis 1·10⁻³ Gew.-%, bevorzugt 0,5·10⁻⁴ Gew.-% bis 9,5·10⁻⁴ Gew.-%,
g) mindestens ein Vitamin B, insbesondere Vitamin B6 und/oder Vitamin B12, in Gesamtmengen von 0,5·10⁻⁶ Gew.-% bis 0,5·10⁻³ Gew.-%, vorzugsweise 0,5·10⁻⁵ Gew.-% bis 5·10⁻⁴ Gew.-%, bevorzugt 1·10⁻⁵ Gew.-% bis 1·10⁻⁴ Gew.-%,
wobei die Mengenangaben jeweils auf die Kauzusammensetzung bezogen sind.

16. Kauzusammensetzung in Form eines Kaugummis nach einem der vorangehenden Ansprüche, insbesondere zur Raucherentwöhnung, enthaltend
a) eine Kaugummigrundmasse in Mengen von 100 mg bis 5.000 mg, insbesondere 200 mg bis 3.000 mg, vorzugsweise 300 mg bis 2.000 mg, bevorzugt 500 mg bis 1.500 mg, besonders bevorzugt 800 mg bis 1.200 mg,
b) einen Kautabak in Mengen von 50 mg bis 1.000 mg, insbesondere 200 mg bis 800 mg, vorzugsweise 300 mg bis 700 mg, bevorzugt 400 mg bis 600 mg,
c) mindestens ein Phytoalexin, bevorzugt Resveratrol, in Mengen von 5 mg bis 400 mg, insbesondere 10 mg bis 300 mg, vorzugsweise 15 mg bis 200 mg, bevorzugt 20 mg bis 100 mg, besonders bevorzugt 30 mg bis 80 mg,
d) Ascorbinsäure (Vitamin C) in Mengen von 5 mg bis 400 mg, insbesondere 10 mg bis 300 mg, vorzugsweise 15 mg bis 200 mg, bevorzugt 20 mg bis 100 mg, besonders bevorzugt 30 mg bis 80 mg,
e) mindestens ein Polyphenol, insbesondere mindestens ein Catechin, vorzugsweise Epicatechin, in Mengen von 1 mg bis 300 mg, insbesondere 5 mg bis 250 mg, vorzugsweise 10 mg bis 200 mg, bevorzugt 15 mg bis 100 mg, besonders bevorzugt 20 mg bis 50 mg,
f) Selen in Mengen von 0,01 µg bis 100 µg, insbesondere 0,1 µg bis 50 µg, vorzugsweise 0,5 µg bis 25 µg, bevorzugt 1 µg bis 20 µg, besonders bevorzugt 1 µg bis 10 µg,
g) mindestens ein Vitamin B, insbesondere Vitamin B6 und/oder Vitamin B12, in Gesamtmengen von 0,005 µg bis 50 µg, insbesondere 0,01 µg bis 20 µg, vorzugsweise 0,05 µg bis 15 µg, bevorzugt 0,1 µg bis 10 µg, besonders bevorzugt 0,5 µg bis 5 µg,
wobei die Mengenangaben jeweils auf eine Dosiereinheit der Kauzusammensetzung, insbesondere auf einen einzelnen Kaugummi, bezogen sind.

## Claims

1. A chewing composition in the form of chewing gum, in particular for smoking cessation, the chewing composition containing in addition to
a) 20% by weight to 95% by weight of a chewing gum main component
b) 5% by weight to 60% by weight of a chewing tobacco containing nicotine,
wherein the chewing composition further contains
c) at least one phytoalexin in amounts of 0.1% by weight to 10% by weight,
d) ascorbic acid (vitamin C) in amounts of 0.1% by weight to 10% by weight,
e) at least one polyphenol in amounts of 0.2% by weight to 10% by weight,
f) selenium in amounts of 1·10⁻⁷% by weight to 1·10⁻²% by weight, and
g) at least one vitamin B in amounts of 0.5·10⁻⁷% by weight to 0.5·10⁻²% by weight,
wherein the above stated amounts are each based on the chewing composition.

2. The chewing composition according to claim 1,
wherein the chewing tobacco is incorporated and/or embedded into the chewing gum main component, and/or
wherein the chewing tobacco is incorporated and/or embedded into the chewing gum main component in an at least essentially homogenous distribution, and/or
wherein the chewing tobacco is incorporated and/or embedded into the chewing gum main component in a crushed and/or finely dispersed manner, particularly pulverized or ground.

3. The chewing composition according to claims 1 or 2, wherein the chewing tobacco has a particle and/or fiber size of 0.001 µm to 5 mm, in particular 0.01 µm to 3 mm, preferably 0.1 µm to 2 mm, most preferably 1 µm to 1 mm.

4. The chewing composition according to one of the preceding claims,
wherein the chewing tobacco has a nicotine content of 0.01% by weight to 2% by weight, in particular 0.05% by weight to 1.5% by weight, preferably 0.1% by weight to 1.25% by weight, preferably 0.2% by weight to 1% by weight, based on the chewing tobacco, and/or
wherein the chewing composition has a nicotine amount of 0.1 mg to 20 mg, in particular 0.5 mg to 15 mg, preferably 0.75 mg to 10 mg, most preferably 1 mg to 5 mg, based on a dosage unit of the chewing composition, in particular based on a single piece of chewing gum.

5. The chewing composition according to one of the preceding claims,
wherein the chewing tobacco has a nitrosamine content of not more than 5 µg of nitrosamine(s)/g of chewing tobacco, in particular not more than 2 µg of nitrosamine(s)/g of chewing tobacco, preferably not more than 1 µg of nitrosamine(s)/g of chewing tobacco, most preferably not more than 0.5 µg of nitrosamine(s)/g of chewing tobacco, in particular preferably not more than 1 µg of nitrosamine(s)/g of chewing tobacco, and in a most preferred manner is at least essentially free of any nitrosamine.

6. The chewing composition according to one of the preceding claims,
wherein the chewing composition comprises the chewing tobacco in amounts of 10% by weight to 50% by weight, preferably 20% by weight to 40% by weight, most preferably 25% by weight to 35% by weight, based on the chewing composition, and/or
wherein the chewing composition comprises the chewing tobacco in amounts of 50 mg to 1,000 mg, in particular 200 mg to 800 mg, preferably 300 mg to 700 mg, most preferably 400 mg to 600 mg, based on a dosage unit of the chewing composition, in particular based on a single piece of chewing gum.

7. The chewing composition according to one of the preceding claims,
wherein the chewing gum main component is essentially water-insoluble, in particular is essentially insoluble in saliva, and/or is essentially indigestible, and/or
wherein the chewing gum main component contains polymers, in particular natural rubber, preferably in the form of latex, crepe, or sheets, and/or polyols, and/or wherein the chewing gum main component contains synthetic polymers, in particular synthetic thermoplasts, preferably polyvinyl ether, in particular poly(ethyl vinyl ether), and/or poly(isobutyl vinyl ether), and/or polyisobutene.

8. The chewing composition according to one of the preceding claims,
wherein the chewing composition comprises the chewing gum main component in amounts of 30% by weight to 90% by weight, preferably 40% by weight to 80% by weight, most preferably 50% by weight to 70% by weight, based on the chewing composition, and/or
wherein the chewing composition comprises the chewing gum main component in amounts of 100 mg to 5,000 mg, in particular 200 mg to 3,000 mg, preferably 300 mg to 2,000 mg, most preferably 500 mg to 1,500 mg, in particular preferably 800 mg to 1,200 mg, based on a dosage unit of the chewing composition, in particular based on a single piece of chewing gum.

9. The chewing composition according to one of the preceding claims,
wherein the chewing composition comprises said phytoalexin, preferably resveratrol, in amounts of 0.5% by weight to 7.5% by weight, preferably 1% by weight to 5% by weight, most preferably 1.5% by weight to 4% by weight, based on the chewing composition, and/or
wherein the chewing composition comprises said phytoalexin, preferably resveratrol, in amounts of 5 mg to 400 mg, in particular 10 mg to 300 mg, preferably 15 mg to 200 mg, most preferably 20 mg to 100 mg, in particular preferably 30 mg to 80 mg, based on a dosage unit of the chewing composition, in particular based on a single piece of chewing gum.

10. The chewing composition according to one of the preceding claims,
wherein the chewing composition comprises said ascorbic acid (vitamin C) in amounts of 0.5% by weight to 7.5% by weight, preferably 1% by weight to 5% by weight, most preferably 1.5% by weight to 4% by weight, based on the chewing composition, and/or wherein the chewing composition comprises said ascorbic acid (vitamin C) in amounts of 5 mg to 400 mg, in particular 10 mg to 300 mg, preferably 15 mg to 200 mg, most preferably 20 mg to 100 mg, in particular preferably 30 mg to 80 mg, based on a dosage unit of the chewing composition, in particular based on a single piece of chewing gum.

11. The chewing composition according to one of the preceding claims,
wherein the chewing composition comprises said polyphenol, in particular catechin, preferably epicatechin, in amounts of 0.3% by weight to 6% by weight, preferably 0.5% by weight to 5% by weight, most preferably 1% by weight to 3% by weight, based on the chewing composition, and/or
wherein the chewing composition comprises said polyphenol, in particular catechin, preferably epicatechin, in amounts of 1 mg to 300 mg, in particular 5 mg to 250 mg, preferably 10 mg to 200 mg, most preferably 15 mg to 100 mg, in particular preferably 20 mg to 50 mg, based on a dosage unit of the chewing composition, in particular based on a single piece of chewing gum.

12. The chewing composition according to one of the preceding claims,
wherein the chewing composition comprises said selenium in amounts of 1·10⁻⁶% by weight to 1·10⁻³ % by weight, preferably 1·10⁻⁵% by weight to 1·10⁻³ % by weight, most preferably 0.5·10⁻⁴% by weight to 9.5·10⁻⁴% by weight, based on the chewing composition, and/or
wherein the chewing composition comprises said selenium in amounts of 0.01 µg to 100 µg, in particular 0.1 µg to 50 µg, preferably 0.5 µg to 25 µg, most preferably 1 µg to 20 µg, in particular preferably 1 µg to 10 µg, based on a dosage unit of the chewing composition, in particular based on a single piece of chewing gum.

13. The chewing composition according to one of the preceding claims,
wherein the chewing composition comprises said vitamin B, in particular vitamin B6, and/or vitamin B12, in total amounts of 0.5·10⁻⁶% by weight to 0.5·10⁻³% by weight, preferably 0.5·10⁻⁵% by weight to 5·10⁻⁴% by weight, most preferably 1·10⁻⁵ % by weight to 1·10⁻⁴% by weight, based on the chewing composition, and/or
wherein the chewing composition comprises said vitamin B, in particular vitamin B6, and/or vitamin B12, in total amounts of 0.005 µg to 50 µg, in particular 0.01 µg to 20 µg, preferably 0.05 µg to 15 µg, most preferably 0.1 µg to 10 µg, in particular preferably 0.5 µg to 5 µg, based on a dosage unit of the chewing composition, in particular based on a single piece of chewing µm.

14. The chewing composition according to one of the preceding claims, further containing at least one additional ingredient that is selected from the group consisting of additives and/or adjuvants, such as fillers, extenders, binders, surfactants, stabilizers, colorants, buffers, scents, flavorings, sweeteners, aromatics, processing aids, and preservatives, as well as the mixtures thereof.

15. The chewing composition in the form of chewing gum according to one of the preceding claims, in particular for smoking cessation, comprising
a) chewing gum main component in amounts of 30% by weight to 90% by weight, preferably 40% by weight to 80% by weight, most preferably 50% by weight to 70% by weight,
b) chewing tobacco in amounts of 10% by weight to 50% by weight, preferably 20% by weight to 40% by weight, preferably 25% by weight to 35% by weight,
c) at least one phytoalexin, preferably resveratrol, in amounts of 0.5% by weight to 7.5% by weight, preferably 1% by weight to 5% by weight, most preferably 1.5% by weight to 4% by weight,
d) ascorbic acid (vitamin C) in amounts of 0.5% by weight to 7.5% by weight, preferably 1% by weight to 5% by weight, most preferably 1.5% by weight to 4% by weight,
e) at least one polyphenol, in particular at least one catechin, preferably epicatechin, in amounts of 0.3% by weight to 6% by weight, preferably 0.5% by weight to 5% by weight, most preferably 1% by weight to 3% by weight,
f) selenium in amounts of 1·10⁻⁷% by weight to 1·10⁻²% by weight, in particular 1·10⁻⁶ % by weight to 1·10⁻³ % by weight, preferably 1·10⁻⁵% by weight to 1·10⁻³ % by weight, most preferably 0.5·10⁻⁴% by weight to 9.5·10⁻⁴% by weight,
g) at least one vitamin B, in particular vitamin B6, and/or vitamin B12, in total amounts of 0.5·10⁻⁶% by weight to 0.5·10⁻³% by weight, preferably 0.5·10⁻⁵% by weight to 5·10⁻⁴ % by weight, most preferably 1·10⁻⁵% by weight to 1·10⁻⁴% by weight,
wherein the amounts are each based on the chewing composition.

16. The chewing composition in the form of chewing gum according to one of the preceding claims, in particular for smoking cessation, comprising
a) chewing gum main component in amounts of 100 mg to 5,000 mg, in particular 200 mg to 3,000 mg, preferably 300 mg to 2,000 mg, most preferably 500 mg to 1,500 mg, in particular preferably 800 mg to 1,200 mg,
b) chewing tobacco in amounts of 50 mg to 1,000 mg, in particular 200 mg to 800 mg, preferably 300 mg to 700 mg, most preferably 400 mg to 600 mg,
c) at least one phytoalexin, preferably resveratrol, in amounts of 5 mg to 400 mg, in particular 10 mg to 300 mg, preferably 15 mg to 200 mg, most preferably 20 mg to 100 mg, in particular preferably 30 mg to 80 mg,
d) ascorbic acid (vitamin C) in amounts of 5 mg to 400 mg, in particular 10 mg to 300 mg, preferably 15 mg to 200 mg, most preferably 20 mg to 100 mg, in particular preferably 30 mg to 80 mg,
e) at least one polyphenol, in particular at least one catechin, preferably epicatechin, in amounts of 1 mg to 300 mg, in particular 5 mg to 250 mg, preferably 10 mg to 200 mg, most preferably 15 mg to 100 mg, in particular preferably 20 mg to 50 mg,
f) selenium in amounts of 0.01 µg to 100 µg, in particular 0.1 µg to 50 µg, preferably 0.5 µg to 25 µg, most preferably 1 µg to 20 µg, in particular preferably 1 µg to 10 µg,
g) at least one vitamin B, in particular vitamin B6, and/or vitamin B12, in total amounts of 0.005 µg to 50 µg, in particular 0.01 µg to 20 µg, preferably 0.05 µg to 15 µg, most preferably 0.1 µg to 10 µg, in particular preferably 0.5 µg to 5 µg,
wherein the amounts are each based on a dosage unit of the chewing composition, in particular on a single piece of chewing gum.

## Revendications

1. Composition à mâcher sous forme d'une gomme à mâcher, en particulier pour le sevrage tabagique, la composition à mâcher contenant, outre
a) 20 % en poids à 95 % en poids d'une base de gomme à mâcher,
b) 5 % en poids à 60 % en poids d'un tabac à chiquer contenant de la nicotine,
la composition à mâcher contenant de plus
c) au moins une phytoalexine en des quantités de 0,1 % en poids à 10 % en poids,
d) de l'acide ascorbique (vitamine C) en des quantités de 0,1 % en poids à 10 % en poids,
e) au moins un polyphénol en des quantités de 0,2 % en poids à 10 % en poids,
f) du sélénium en des quantités de 1.10⁻⁷ % en poids à 1.10⁻² % en poids, et
g) au moins une vitamine B en des quantités de 0,5·10⁻⁷ % en poids à 0,5·10⁻² % en poids,
chacune des indications de quantité étant rapportée à la composition à mâcher.

2. Composition à mâcher selon la revendication 1,
dans laquelle le tabac à chiquer est incorporé et/ou enrobé dans la base de gomme à mâcher, et/ou
dans laquelle le tabac à chiquer est incorporé et/ou enrobé dans la masse de gomme à mâcher selon une répartition au moins essentiellement homogène, et/ou
dans laquelle le tabac à chiquer est incorporé et/ou enrobé dans la masse de gomme à mâcher sous une forme broyée et/ou finement divisée, en particulier pulvérisé ou moulu.

3. Composition à mâcher selon la revendication 1 ou 2, dans laquelle le tabac à chiquer présente une granulométrie et/ou une grosseur des fibres de 0,001 µm à 5 mm, en particulier de 0,01 µm à 3 mm, de préférence de 0,1 µm à 2 mm, d'une manière préférée de 1 µm à 1 mm.

4. Composition à mâcher selon l'une des revendications précédentes,
dans laquelle le tabac à chiquer présente une teneur en nicotine de 0,01 % en poids à 2 % en poids, en particulier de 0,05 % en poids à 1,5 % en poids, de préférence de 0,1 % en poids à 1,25 % en poids, d'une manière préférée de 0,2 % en poids à 1 % en poids, par rapport au tabac à chiquer, et/ou
la composition à mâcher contenant une quantité de nicotine de 0,1 mg à 20 mg, en particulier de 0,5 mg à 15 mg, de préférence de 0,75 mg à 10 mg, d'une manière préférée de 1 mg à 5 mg, rapportée à une unité posologique de la composition à mâcher, en particulier à une gomme à mâcher individuelle.

5. Composition à mâcher selon l'une des revendications précédentes,
dans laquelle le tabac à chiquer présente une teneur en nitrosamine d'au plus 5 µg de nitrosamine(s)/g de tabac à chiquer, en particulier au plus 2 µg de nitrosamine(s)/g de tabac à chiquer, de préférence au plus 1 µg de nitrosamine(s)/g de tabac à chiquer, d'une manière préférée au plus 0,5 µg de nitrosamine(s)/g de tabac à chiquer, d'une manière particulièrement préférée au plus 1 µg de nitrosamine(s)/g de tabac à chiquer, et d'une manière tout particulièrement préférée est au moins essentiellement exempt de nitrosamine.

6. Composition à mâcher selon l'une des revendications précédentes,
la composition à mâcher contenant le tabac à chiquer en des quantités de 10 % en poids à 50 % en poids, de préférence de 20 % en poids à 40 % en poids, d'une manière préférée de 25 % en poids à 35 % en poids, par rapport à la composition à mâcher, et/ou
la composition à mâcher contenant une quantité de tabac à chiquer de 50 mg à 1 000 mg, en particulier de 200 mg à 800 mg, de préférence de 300 mg à 700 mg, d'une manière préférée de 400 mg à 600 mg, rapportées à une unité posologique de la composition à mâcher, en particulier à une gomme à mâcher individuelle.

7. Composition à mâcher selon l'une des revendications précédentes, dans laquelle la base de gomme à mâcher est au moins essentiellement insoluble dans l'eau, en particulier au moins essentiellement insoluble dans la salive, et/ou au moins essentiellement indigestible, et/ou
dans laquelle la base de gomme à mâcher contient des polymères naturels, en particulier du caoutchouc, de préférence sous forme de latex, de crêpe ou de feuilles, et/ou des polyols, et/ou
dans laquelle la base de gomme à mâcher contient des polymères synthétiques, en particulier des thermoplastiques synthétiques, de préférence du polyvinyléther, en particulier du poly(éthylvinyléther) et/ou du poly(isobutylvinyléther) et/ou des polyisobutènes.

8. Composition à mâcher selon l'une des revendications précédentes,
la composition à mâcher contenant la base de gomme à mâcher en des quantités de 30 % en poids à 90 % en poids, de préférence de 40 % en poids à 80 % en poids, d'une manière préférée de 50 % en poids à 70 % en poids, par rapport à la composition à mâcher, et/ou
la composition à mâcher contenant la base de gomme à mâcher en des quantités de 100 mg à 5 000 mg, en particulier de 200 mg à 3 000 mg, de préférence de 300 mg à 2 000 mg, d'une manière préférée de 500 mg à 1 500 mg, d'une manière particulièrement préférée de 800 mg à 1 200 mg, rapportées à une unité posologique de la composition à mâcher, en particulier à une gomme à mâcher individuelle.

9. Composition à mâcher selon l'une des revendications précédentes,
la composition à mâcher contenant la phytoalexine, de préférence le resvératrol, en des quantités de 0,5 % en poids à 7,5 % en poids, de préférence de 1 % en poids à 5 % en poids, d'une manière préférée de 1,5 % en poids à 4 % en poids, par rapport à la composition à mâcher, et/ou
la composition à mâcher contenant la phytoalexine, de préférence le resvératrol, en des quantités de 5 mg à 400 mg, en particulier de 10 mg à 300 mg, de préférence de 15 mg à 200 mg, d'une manière préférée de 20 mg à 100 mg, d'une manière particulièrement préférée de 30 mg à 80 mg, rapportées à une unité posologique de la composition à mâcher, en particulier à une gomme à mâcher individuelle.

10. Composition à mâcher selon l'une des revendications précédentes,
la composition à mâcher contenant l'acide ascorbique (vitamine C) en des quantités de 0,5 % en poids à 7,5 % en poids, de préférence de 1 % en poids à 5 % en poids, d'une manière préférée de 1,5 % en poids à 4 % en poids, par rapport à la composition à mâcher, et/ou
la composition à mâcher contenant l'acide ascorbique (vitamine C) en des quantités de 5 mg à 400 mg, en particulier de 10 mg à 300 mg, de préférence de 15 mg à 200 mg, d'une manière préférée de 20 mg à 100 mg, d'une manière particulièrement préférée de 30 mg à 80 mg, rapportées à une unité posologique de la composition à mâcher, en particulier à une gomme à mâcher individuelle.

11. Composition à mâcher selon l'une des revendications précédentes,
la composition à mâcher contenant le polyphénol, en particulier la catéchine, de préférence l'épicatéchine, en des quantités de 0,3 % en poids à 6 % en poids, de préférence de 0,5 % en poids à 5 % en poids, d'une manière préférée de 1 % en poids à 3 % en poids, par rapport à la composition à mâcher, et/ou
la composition à mâcher contenant le polyphénol, en particulier la catéchine, de préférence l'épicatéchine, en des quantités de 1 mg à 300 mg, en particulier de 5 mg à 250 mg, de préférence de 10 mg à 200 mg, d'une manière préférée de 15 mg à 100 mg, d'une manière particulièrement préférée de 20 mg à 50 mg, rapportées à une unité posologique de la composition à mâcher, en particulier à une gomme à mâcher individuelle.

12. Composition à mâcher selon l'une des revendications précédentes,
la composition à mâcher contenant le sélénium en des quantités de 1.10⁻⁶ % en poids à 1.10⁻³ % % en poids, de préférence de 1.10⁻⁵ % en poids à 1.10⁻³ % en poids, d'une manière préférée de 0,5.10⁻⁴ % en poids à 9,5.10⁻⁴ % en poids, par rapport à la composition à mâcher, et/ou
la composition à mâcher contenant le sélénium en des quantités de 0,01 µg à 100 µg, en particulier de 0,1 µg à 50 µg, de préférence de 0,5 µg à 25 µg, d'une manière préférée de 1 µg à 20 µg, d'une manière particulièrement préférée de 1 µg à 10 µg, rapportées à une unité posologique de la composition à mâcher, en particulier à une gomme à mâcher individuelle.

13. Composition à mâcher selon l'une des revendications précédentes,
la composition à mâcher contenant la vitamine B, en particulier la vitamine B6 et/ou la vitamine B12, en des quantités totales de 0,5.10⁻⁶ % en poids à 0,5.10⁻³ % en poids, de préférence de 0,5.10⁻⁵ % en poids à 5.10⁻⁴ % en poids, d'une manière préférée de 1.10⁻⁵ % en poids à 1.10⁻⁴ % en poids, par rapport à la composition à mâcher, et/ou
la composition à mâcher contenant la vitamine B, en particulier la vitamine B6 et/ou la vitamine B12, en des quantités totales de 0,005 µg à 50 µg, en particulier de 0,01 µg à 20 µg, de préférence de 0,05 µg à 15 µg, d'une manière préférée de 0,1 µg à 10 µg, d'une manière particulièrement préférée de 0,5 µg à 5 µg, rapportées à une unité posologique de la composition à mâcher, en particulier à une gomme à mâcher individuelle.

14. Composition à mâcher selon l'une des revendications précédentes, contenant en outre au moins un constituant supplémentaire choisi dans le groupe des additifs et/ou adjuvants, tels que les substances et agents servant de matières de charge, de diluants, de liants, de mouillants, de stabilisants, de colorants, de tampons, de parfums, d'agents de sapidité, d'édulcorants, d'aromatisants, d'auxiliaires de mise en oeuvre et de conservateurs.

15. Composition à mâcher sous forme d'une gomme à mâcher selon l'une des revendications précédentes, en particulier pour le sevrage tabagique, contenant
a) une base de gomme à mâcher en des quantités de 30 % en poids à 90 % en poids, de préférence de 40 % en poids à 80 % en poids, d'une manière préférée de 50 % en poids à 70 % en poids,
b) un tabac à chiquer en des quantités de 10 % en poids à 50 % en poids, de préférence de 20 % en poids à 40 % en poids, d'une manière préférée de 25 % en poids à 35 % en poids,
c) au moins une phytoalexine, de préférence le resvératrol, en des quantités de 0,5 % en poids à 7,5 % en poids, de préférence de 1 % en poids à 5 % en poids, d'une manière préférée de 1,5 % en poids à 4 % en poids,
d) de l'acide ascorbique (vitamine C) en des quantités de 0,5 % en poids à 7,5 % en poids, de préférence de 1 % en poids à 5 % en poids, d'une manière préférée de 1,5 % en poids à 4 % en poids,
e) au moins un polyphénol, en particulier au moins une catéchine, de préférence l'épicatéchine, en des quantités de 0,3 % en poids à 6 % en poids, de préférence de 0,5 % en poids à 5 % en poids, d'une manière préférée de 1 % en poids à 3 % en poids,
f) du sélénium en des quantités de 1.10⁻⁷ % en poids à 1.10⁻² % en poids, en particulier de 1.10⁻⁶ % en poids à 1.10⁻³ % en poids, de préférence de 1.10⁻⁵ % en poids à 1.10⁻³ % en poids, d'une manière préférée de 0,5.10⁻⁴ % en poids à 9,5.10⁻⁴ % en poids,
g) au moins une vitamine B, en particulier la vitamine B6 et/ou la vitamine B12, en des quantités totales de 0,5.10⁻⁶ % en poids à 0,5.10⁻³ % % en poids, de préférence de 0,5.10⁻⁵ % en poids à 5.10⁻⁴ % en poids, d'une manière préférée de 1.10⁻⁵ % en poids à 1.10⁻⁴ % en poids,
chacune des indications de quantité étant rapportée à la composition à mâcher.

16. Composition à mâcher sous forme d'une gomme à mâcher selon l'une des revendications précédentes, en particulier pour le sevrage tabagique, contenant
a) une masse de gomme à mâcher en des quantités de 100 mg à 5 000 mg, en particulier de 200 mg à 3 000 mg, de préférence de 300 mg à 2 000 mg, d'une manière préférée de 500 mg à 1 500 mg, d'une manière particulièrement préférée de 800 mg à 1 200 mg,
b) un tabac à chiquer en des quantités de 50 mg à 1 000 mg, en particulier de 200 mg à 800 mg, de préférence de 300 mg à 700 mg, d'une manière préférée de 400 mg à 600 mg,
c) au moins une phytoalexine, de préférence le resvératrol, en des quantités de 5 mg à 400 mg, en particulier de 10 mg à 300 mg, de préférence de 15 mg à 200 mg, d'une manière préférée de 20 mg à 100 mg, d'une manière particulièrement préférée de 30 mg à 80 mg,
d) de l'acide ascorbique (vitamine C) en des quantités de 5 mg à 400 mg, en particulier de 10 mg à 300 mg, de préférence de 15 mg à 200 mg, d'une manière préférée de 20 mg à 100 mg, d'une manière particulièrement préférée de 30 mg à 80 mg,
e) au moins un polyphénol, en particulier au moins une catéchine, de préférence l'épicatéchine, en des quantités de 1 mg à 300 mg, en particulier de 5 mg à 250 mg, de préférence de 10 mg à 200 mg, d'une manière préférée de 15 mg à 100 mg, d'une manière particulièrement préférée de 20 mg à 50 mg,
f) du sélénium en des quantités de 0,01 µg à 100 µg, en particulier de 0,1 µg à 50 µg, de préférence de 0,5 µg à 25 µg, d'une manière préférée de 1 µg à 20 µg, d'une manière particulièrement préférée de 1 µg à 10 µg,
g) au moins une vitamine B, en particulier la vitamine B6 et/ou la vitamine B12, en des quantités totales de 0,005 µg à 50 µg, en particulier de 0,01 µg à 20 µg, de préférence de 0,05 µg à 15 µg, d'une manière préférée de 0,1 µg à 10 µg, d'une manière particulièrement préférée de 0,5 µg à 5 µg,
chacune des indications de quantité étant rapportée à une unité posologique de la composition à mâcher, en particulier à une gomme à mâcher individuelle.
